(19) Europäisches Patentamt
European Patent Office
Office européen des brevets

(11) EP 4 374 874 A1

(12) **EUROPEAN PATENT APPLICATION**
published in accordance with Art. 153(4) EPC

(43) Date of publication:
**29.05.2024 Bulletin 2024/22**

(21) Application number: **22845411.2**

(22) Date of filing: **21.07.2022**

(51) International Patent Classification (IPC):
***A61K 39/395*** *(2006.01)* ***C07K 16/30*** *(2006.01)*
***A61P 35/00*** *(2006.01)*

(52) Cooperative Patent Classification (CPC):
**A61K 47/68037; A61K 39/39591; A61K 47/6851; A61K 47/6889; A61P 35/00; C07K 16/30;**
A61K 2039/505; C07K 2317/77; C07K 2317/92; C07K 2319/55

(86) International application number:
**PCT/CN2022/107134**

(87) International publication number:
**WO 2023/001248 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.07.2021 CN 202110823295**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **MO, Xiyele**
**Shanghai 200245 (CN)**
- **TIAN, Chenmin**
**Shanghai 200245 (CN)**
- **LIU, Xun**
**Shanghai 200245 (CN)**

(74) Representative: **Dragotti & Associati S.R.L.**
**Via Nino Bixio, 7**
**20129 Milano (IT)**

Remarks:
The complete document including Reference Table(s) and the Sequence Listing(s) can be downloaded from the EPO website

(54) **PHARMACEUTICAL COMPOSITION CONTAINING ANTI-TROP2 ANTIBODY DRUG CONJUGATE AND USE THEREOF**

(57) The disclosure relates to a pharmaceutical composition containing an anti-TROP2 antibody drug conjugate and its application. Specifically, the disclosure relates to a pharmaceutical composition comprising an antibody drug conjugate in a buffer. The pharmaceutical composition has good stability.

EP 4 374 874 A1

## Description

### TECHNICAL FIELD

The present disclosure belongs to the field of pharmaceutical formulations, and in particular relates to a pharmaceutical composition comprising an anti-TROP2 antibody drug conjugate and use thereof as an anti-cancer medicament.

### BACKGROUND

The statements herein merely provide background information related to the present disclosure and may not necessarily constitute the prior art.

Antibody drug conjugate (ADC) links a monoclonal antibody or an antibody fragment to a biologically active cytotoxin via a stable chemical linker compound, fully exploiting the binding specificity of the antibody to surface antigens of tumor cells and the high-efficiency of the cytotoxin, and also avoiding the former's disadvantage of having a poor therapeutic effect, the latter's disadvantage of having serious toxic side effects, and the like. This means that the antibody drug conjugate can bind to tumor cells more precisely and has a reduced effect on normal cells compared to conventional chemotherapeutic drugs in the past (Mullard A, (2013) Nature Reviews Drug Discovery, 12:329-332; DiJoseph JF, Annellino DC, (2004) Blood, 103:1807-1814).

There are several classes of small molecules with cytotoxicity for antibody drug conjugates, one of which is camptothecin derivatives having an antitumor effect by inhibiting topoisomerase I. The camptothecin derivative, irinotecan (chemical name: (1S, 9*S*)-1-amino-9-ethyl-5-fluoro-2,3-dihydro-9-hydroxy-4-methyl-1*H*,12*H*-benzo[*de*]pyrano[3',4':6,7]imidazo[1,2-*b*]quinoline-10,13(9*H*,15*H*)-dione), was reported for use in antibody drug conjugates (ADCs) as described in WO2014057687; Clinical Cancer Research (2016)22 (20):5097-5108; Cancer Sci (2016) 107: 1039-1046. There is still a need to further develop ADC drugs with better therapeutic effects.

However, ADCs are of more complicated heterostructures than antibodies, and therefore, a greater challenge has been posed to ADC formulations for therapeutic purposes.

### SUMMARY

The present disclosure provides a pharmaceutical composition comprising an antibody drug conjugate and a buffer, wherein the antibody drug conjugate has a structure shown below:

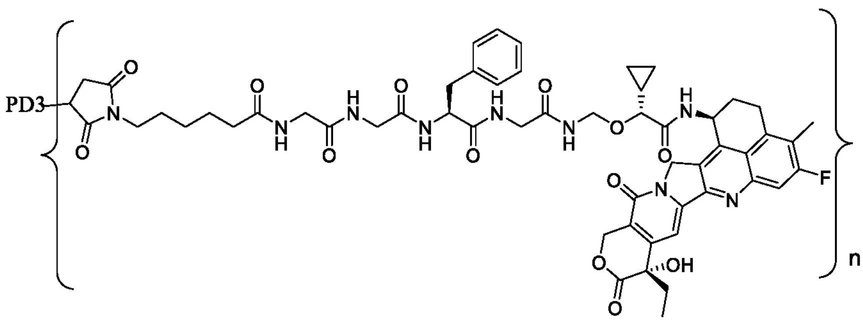

;

The antibody drug conjugate described above is prepared according to Example 3-1 ADC-1 (an example for antibody conjugates) on page 41 of the description of PCT/CN2021/073279;
wherein:
PD3 is an anti-TROP2 antibody prepared according to the antibody PD3 on page 41 of PCT/CN2021/073279 and comprising a heavy chain set forth in SEQ ID NO: 1 (SEQ ID NO: 13 in PCT/CN2021/073279) and a light chain set forth in SEQ ID NO: 2 (SEQ ID NO: 14 in PCT/CN2021/073279):

Heavy chain amino acid sequence of PD3:

EVQLVQSGSELKKPGASVKVSCKASGYTFTNYGMNWVKQAPGQGLKWMGWI
NTYTGEPTYTQDFKGRFAFSLDTSVSTAYLQISSLKAEDTAVYYCARGGFGSSY
WYFDVWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK
SEQ ID NO: 1;

SEQ ID NO: 1;
Light chain amino acid sequence of PD3:

DIQLTQSPSSLSASVGDRVSITCKASQDVSIAVAWYQQKPGKAPKLLIYSASYRY
TGVPDRFSGSGSGTDFTLTISSLQPEDFAVYYCQQHYITPLTFGAGTKVEIKRTVA
APSVFIFPPSDEQLKSGTASVVCLLNNFYPREAKVQWKVDNALQSGNSQESVTE

QDSKDSTYSLSSTLTLSKADYEKHKVYACEVTHQGLSSPVTKSFNRGEC
SEQ ID NO: 2;

n is 1 to 10, preferably 1 to 8, more preferably 3 to 5, and most preferably about 4;
the buffer of the pharmaceutical composition is a histidine-histidine hydrochloride buffer with a pH of about 5.0 to about 6.5, preferably a pH of about 5.5 to about 6.5, and more preferably a pH of about 5.9 to about 6.2.

In an alternative embodiment, the buffer in the pharmaceutical composition has a pH of about 5.0 to about 6.5, and non-limiting examples include about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, and any range therebetween; the pH is preferably about 5.5 to about 6.5 and more preferably about 5.9 to about 6.2.

In alternative embodiment, the buffer in the pharmaceutical composition is at a concentration of about 5 mM to about 50 mM, and non-limiting examples include 10 mM, 12 mM, 15 mM, 16 mM, 17 mM, 18 mM, 19 mM, 20 mM, 30 mM, 40 mM, 50 mM, and any range therebetween; the buffer is preferably at a concentration of about 10 mM to about 50 mM, more preferably about 20 mM to about 40 mM, and most preferably about 30 mM.

In an alternative embodiment, the pharmaceutical composition further comprises a surfactant. The surfactant may be selected from the group consisting of polysorbate, polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. The surfactant is preferably polysorbate 80 or polysorbate 20, and more preferably polysorbate 80.

In an alternative embodiment, the surfactant in the pharmaceutical composition is at a concentration of about 0.01 mg/mL to about 1.0 mg/mL, preferably about 0.05 mg/mL to about 0.5 mg/mL, more preferably about 0.1 mg/mL

to about 0.3 mg/mL or about 0.2 mg/mL to about 0.6 mg/mL, and most preferably about 0.2 mg/mL, and non-limiting examples include about 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.6 mg/mL, 0.7 mg/mL, 0.8 mg/mL, and any range therebetween.

In an alternative embodiment, the aforementioned pharmaceutical composition further comprises a saccharide. "Saccharide" of the present disclosure includes the general composition $(CH_2O)_n$ and derivatives thereof, including monosaccharides, disaccharides, trisaccharides, polysaccharides, sugar alcohols, reducing sugars, non-reducing sugars, etc. The saccharide may be selected from the group consisting of glucose, sucrose, trehalose, lactose, fructose, maltose, dextran, glycerin, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc. The saccharide is preferably non-reducing disaccharide, is more preferably selected from the group consisting of trehalose dihydrate and sucrose, and is most preferably sucrose.

In an alternative embodiment, the saccharide in the aforementioned pharmaceutical composition is at a concentration of about 25 mg/mL to about 80 mg/mL and preferably about 30 mg/mL to about 50mg/mL, and non-limiting examples include 25 mg/mL, 30 mg/mL, 35 mg/mL, 40 mg/mL, 45 mg/mL, 50 mg/mL, 55 mg/mL, 60 mg/mL, 70 mg/mL, 80 mg/mL, and any range therebetween; the saccharide is preferably at a concentration of 40 mg/mL.

In an alternative embodiment, the aforementioned pharmaceutical composition further comprises an amino acid and a salt thereof, and the amino acid or the amino salt thereof is preferably selected from the group consisting of glycine and arginine hydrochloride and is more preferably glycine.

In an alternative embodiment, the glycine in the aforementioned pharmaceutical composition is at a concentration of about 6 mg/mL to about 15 mg/mL, about 7 mg/mL to about 11 mg/mL, preferably about 7 mg/mL to about 10 mg/mL, and more preferably about 7 mg/mL to about 9 mg/mL or about 8 mg/mL to about 9 mg/mL, and non-limiting examples include about 6 mg/mL, 6.5 mg/mL, 7 mg/mL, 7.2 mg/mL, 7.6 mg/mL, 7.8 mg/mL, 8 mg/mL, 8.5 mg/mL, 9 mg/mL, 10 mg/mL, 10.2 mg/mL, 11 mg/mL, 12 mg/mL, 13 mg/mL, 14 mg/mL, 15 mg/mL, and any range therebetween; the glycine is most preferably at a concentration of about 9 mg/mL.

In an alternative embodiment, the antibody drug conjugate in the pharmaceutical composition is at a protein concentration of about 1 mg/mL to about 100 mg/mL, and non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, 100 mg/mL, and any range therebetween; the antibody drug conjugate is preferably at a protein concentration of about 10 mg/mL to about 30 mg/mL, more preferably about 18 mg/mL to about 22 mg/mL, and most preferably about 20 mg/mL. Specifically, non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL, 21 mg/mL, and any range therebetween. The protein concentration refers to the concentration of the antibody moiety in the antibody drug conjugate. In an alternative embodiment, the range of drug loading (n) may be an average number of cytotoxic drugs bound per anti-Trop2 antibody, and non-limiting examples include an average number of cytotoxic drugs bound per antibody of about 1, 2, 3, 4, 5, 6, 7, 8, 9, 10, 11, 12, and any range therebetween. Preferably, the drug loading is selected from the group consisting of 2-8, 2-7, 2-6, 2-5, 2-4, 3-4, 3-5, 5-6, 5-7, 5-8, and 6-8. Illustratively, the drug loading may be an average number of 1, 2, 3, 4, 5, 6, 7, 8, 9, and 10. n is a decimal or an integer.

In an alternative embodiment, the drug loading (n) is about 4.

In an alternative embodiment, the pharmaceutical composition comprises:

(a) the antibody drug conjugate at a protein concentration of about 10 mg/mL to about 30 mg/mL, (b) about 0.1 mg/mL to about 0.3 mg/mL polysorbate, (c) about 30 mg/mL to about 50 mg/mL saccharide, (d) about 7 mg/mL to about 11 mg/mL glycine, and (e) about 20 mM to about 40 mM histidine-histidine hydrochloride buffer, the composition having a pH of about 5.5-6.5;

preferably, the pharmaceutical composition comprises the following components:

(a) the antibody drug conjugate at a protein concentration of about 18 mg/mL to about 22 mg/mL, (b) about 0.2 mg/mL polysorbate 80, (c) about 40 mg/mL sucrose, (d) about 9 mg/mL glycine, and (e) about 30 mM histidine-histidine hydrochloride buffer, the composition having a pH of about 5.9-6.2;

more preferably, the pharmaceutical composition comprises the following components:

(a) the antibody drug conjugate at a protein concentration of about 20 mg/mL, (b) about 0.2 mg/mL polysorbate 80, (c) about 40 mg/mL sucrose, (d) about 9 mg/mL glycine, and (e) about 30 mM histidine-histidine hydrochloride buffer, the composition having a pH of about 6.0.

In an alternative embodiment, the pharmaceutical composition of any one of the aforementioned embodiments

is a liquid formulation. The liquid formulation or the reconstituted formulation of the present invention has relatively good stability and can be stored at the refrigeration temperature (4 °C) for at least 3 months. In addition, stable liquid formulations include liquid formulations that exhibit desired features after storage at temperatures including 25 °C for periods including 1 month, 2 months, and 3 months. Further, stable liquid formulations include liquid formulations that exhibit desired features after storage at temperatures including 40 °C for 1 month.

The present disclosure further provides a lyophilized formulation comprising an antibody drug conjugate, wherein the formulation can be reconstituted to form the pharmaceutical composition described above.

The present disclosure further provides a method for preparing a lyophilized formulation comprising an antibody drug conjugate, which comprises a step of lyophilizing the pharmaceutical composition described above.

In an alternative embodiment, the lyophilizing in the method for preparing a lyophilized formulation comprising an antibody drug conjugate comprises steps of pre-freezing, primary drying, and secondary drying in sequence. The lyophilizing is performed by freezing the formulation and subsequently sublimating the water at a temperature suitable for primary drying. Under these conditions, the product is at a temperature lower than the eutectic point or the collapse temperature of the formulation. Typically, the temperature for the primary drying ranges from about -30 °C to 25 °C (assuming the product remains frozen during the primary drying). The formulation, the size and type of the container (e.g., glass vial) containing the sample, and the liquid volume determine the time required for drying, which may range from a few hours to several days (e.g., 40-60 hours). The secondary drying may be performed at about 0-40 °C, which mainly depends on the type and size of the container and the type of the protein used. The time needed for the secondary drying is determined by the desired residual moisture content in the product, and it typically takes at least about 5 hours. Generally, the water content of the formulation lyophilized at low pressure is less than about 5%, preferably less than about 3%. The pressure may be the same as that applied to the step of primary drying; preferably, the pressure of the secondary drying is lower than that of the primary drying. The lyophilization conditions may vary with the formulation and vial size.

In an alternative embodiment of the present disclosure, 4.4 mL of a stock solution of the composition is lyophilized, and the lyophilization procedure is as follows: pre-freezing at temperatures of 5 °C and -45 °C successively; primary drying at a temperature of -20 °C and at a degree of vacuum of 10 Pa; and secondary drying at a temperature of 25 °C and at degrees of vacuum of 10 Pa and 1 Pa successively.

In some embodiments, the lyophilized formulation is stable at 2-8 °C for at least 16 days, at least 1 month, at least 3 months, at least 6 months, at least 12 months, at least 18 months, or at least 24 months. In some embodiments, the lyophilized formulation is stable at 40 °C for at least 7 days, at least 14 days, at least 28 days, or at least 30 days. The present disclosure further provides a lyophilized formulation comprising an antibody drug conjugate obtained by lyophilizing the pharmaceutical composition of the antibody drug conjugate described above.

The present disclosure further provides a reconstituted solution comprising an antibody drug conjugate, wherein the reconstituted solution is prepared by reconstituting the lyophilized formulation described above.

The present disclosure further provides a method for preparing the reconstituted solution described above, which comprises a step of reconstituting the aforementioned lyophilized formulation with a solution selected from the group consisting of, but not limited to, water for injection, normal saline, and glucose solution.

In an alternative embodiment, the reconstituted solution comprises the following components:

(a) about 10 mg/mL to about 30 mg/mL antibody drug conjugate, (b) about 0.1 mg/mL to about 0.3 mg/mL polysorbate, (c) about 25 mg/mL to about 80 mg/mL sucrose, (d) about 7 mg/mL to about 11 mg/mL glycine, and (e) about 10 mM to about 40 mM histidine-histidine hydrochloride buffer, the composition having a pH of about 5.5-6.5;
preferably, the pharmaceutical composition comprises the following components:

(a) about 18 mg/mL to about 22 mg/mL antibody drug conjugate, (b) about 0.2 mg/mL polysorbate 80, (c) about 40 mg/mL sucrose, (d) about 9 mg/mL glycine, and (e) about 30 mM histidine-histidine hydrochloride, the composition having a pH of about 5.9-6.2; more preferably, the pharmaceutical composition comprises the following components:
(a) about 20 mg/mL antibody drug conjugate, (b) about 0.2 mg/mL polysorbate 80, (c) about 40 mg/mL sucrose, (d) about 9 mg/mL glycine, and (e) about 30 mM histidine-histidine hydrochloride, the composition having a pH of about 6.0.

The present disclosure further provides an article of manufacture comprising a container containing the pharmaceutical composition, the lyophilized formulation or the reconstituted solution described above. In some embodiments, the container is a tubular injection vial made of neutral borosilicate glass.

The present disclosure further provides use of the aforementioned pharmaceutical composition, lyophilized formulation, reconstituted solution, or article of manufacture in preparing a medicament for treating or preventing a tumor.

The present disclosure further provides a method for treating a disease comprising providing the aforementioned pharmaceutical composition, lyophilized formulation, reconstituted solution, or article of manufacture.

The present disclosure further provides use of the aforementioned pharmaceutical composition, lyophilized formulation, reconstituted solution, or article of manufacture as a medicament in the treatment of a disease.

The present disclosure further provides the aforementioned pharmaceutical composition, lyophilized formulation, reconstituted solution, or article of manufacture as a medicament, which is preferably used for treating or preventing a tumor disease.

In an alternative embodiment, the disease or tumor is a TROP2-mediated disease or condition.

The present disclosure further provides use of the aforementioned pharmaceutical composition, lyophilized formulation, reconstituted solution, or article of manufacture as a medicament in preparing a medicament for the treatment and/or prevention of a tumor and cancer, wherein the tumor and the cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing’s sarcoma, urothelium carcinoma, and Merkel cell carcinoma; more preferably, the lymphoma is selected from the group consisting of Hodgkin’s lymphoma, non-Hodgkin’s lymphoma, diffuse large B-cell lymphoma, follicular lymphoma, primary mediastinal large B-cell lymphoma, mantle cell lymphoma, small lymphocytic lymphoma, T-cell/histiocyte-rich large B-cell lymphoma, and lymphoplasmacytic lymphoma; the lung cancer is selected from the group consisting of non-small cell lung cancer and small cell lung cancer; the leukemia is selected from the group consisting of chronic myeloid leukemia, acute myeloid leukemia, lymphocytic leukemia, lymphoblastic leukemia, acute lymphoblastic leukemia, chronic lymphocytic leukemia, and myeloid cell leukemia.

As is well known to those skilled in the art, one, some or all of the features of the various embodiments described in the present disclosure may be further combined to form other embodiments of the present disclosure. The above embodiments of the present disclosure and other embodiments obtained by combination are further illustrated through the following detailed description.

## Detailed Description of the Invention

The present disclosure provides a pharmaceutical composition that favors production and administration and is stable in properties. Undesirable instability may include any one or more of aggregation, deamidation (e.g., Asn deamidation), oxidation (e.g., Met oxidation), isomerization (e.g., Asp isomerization), clipping/hydrolysis/fragmentation (e.g., fragmentation of hinge region), formation of succinimide, unpaired cysteines, dissociation of toxins, and the like. In particular, the pharmaceutical composition described in the present disclosure comprises an antibody drug conjugate and a buffer.

## Terminology

In order to facilitate the understanding of the present disclosure, some technical and scientific terms are specifically defined below. Unless otherwise explicitly defined herein, all other technical and scientific terms used herein have the meanings generally understood by those of ordinary skill in the art to which the present disclosure belongs. The content of the application WO2020063673 is incorporated herein by reference in its entirety.

"Antibody drug conjugate (ADC)" means that an antibody is linked to a biologically active cytotoxin or a small-molecule drug with cell killing activity by a linker unit.

"Drug loading", also referred to as drug-to-antibody ratio (DAR), refers to the average number of drugs conjugated to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 3-4, 3-5, 5-6, 5-7, 5-8 and 6-8 drugs conjugated to each antibody. Illustratively, the drug loading may be an average number of 1, 2, 3, 4, 5, 6, 7, 8, 9, and 10. The ADC general formulas of the present disclosure include a group of antibodies conjugated to drugs within a certain range as described above. In embodiments of the present disclosure, the drug loading may be represented as n. Drug loading can be determined by conventional methods such as UV/visible spectroscopy, mass spectrometry, ELISA assay and HPLC.

The term "linker unit" or "linker fragment" refers to a chemical structure fragment or bond, which is linked to an antibody or antigen-binding fragment thereof at one end and to a drug at the other end, and also may be linked to a drug after being linked to another linker.

The linker includes a stretcher unit, a spacer unit and an amino acid unit, and may be synthesized by methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research,

52: 127-131(1992); U.S. Patent No. 5,208,020).

The loading of the cytotoxic drug can be controlled using the following non-limiting methods, including:

(1) controlling a molar ratio of a linking reagent to a monoclonal antibody,
(2) controlling reaction time and temperature, and
(3) selecting different reaction reagents.

The three-letter and single-letter codes for amino acids used in the present disclosure are as described in J. Biol. Chem., 243, p3558 (1968).

The "antibody" described herein is used in the broadest sense and encompasses a variety of antibody structures, including but not limited to full-length antibodies, and antibody fragments (or antigen-binding fragments, or antigen-binding moieties) so long as they exhibit the desired antigen-binding activity. In general, a natural intact antibody is of a tetrapeptide chain structure formed by connection between two identical heavy chains and two identical light chains by interchain disulfide bonds.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Buffer" refers to a buffer that resists changes in pH by the action of its acid-base conjugate components. Examples of buffers that control the pH in an appropriate range include acetate, succinate, gluconate, histidine, oxalate, lactate, phosphate, citrate, tartrate, fumarate, glycylglycine and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like, and the histidine-histidine hydrochloride buffer is preferred. The histidine-histidine hydrochloride buffer can be prepared from histidine and hydrochloric acid or histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared with succinic acid and sodium hydroxide or with succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Pharmaceutical composition" refers to a mixture containing one or more of the antibody drug conjugates described herein or physiologically/pharmaceutically acceptable salts or prodrugs thereof, and other chemical components, wherein the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "formulation" are not mutually exclusive.

Unless otherwise stated, the solvent in the pharmaceutical composition described herein in solution form is water.

"Lyophilized formulation" refers to a formulation or a pharmaceutical composition obtained by lyophilizing a pharmaceutical composition or a formulation in liquid or solution form in vacuum.

The terms "about" and "approximately" as used herein mean that a numerical value is within an acceptable

error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise stated, when a specific value is provided in the present application and claims, the meaning of "about" or "substantially comprising" should be assumed to be within an acceptable error range for that specific value.

The pharmaceutical composition of the present disclosure can achieve a stable effect: a pharmaceutical composition in which the antibody drug conjugate substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

Stable formulations are formulations in which no significant change is observed under the following conditions: storage at the refrigeration temperature (2-8 °C) for at least 3 months, preferably 6 months, and more preferably 1 year. In addition, stable liquid formulations include liquid formulations that exhibit desired features after storage at temperatures including 25 °C for periods including 1 month, 2 months, and 3 months. Further, stable liquid formulations include liquid formulations that exhibit desired features after storage at temperatures including 40 °C for periods including 10 days, 20 days, and 1 month. Typical examples for stability are as follows: generally, no more than about 10%, preferably no more than about 5%, of antibody monomers aggregate or are degraded as measured by SEC-HPLC. The formulation is a pale yellow, nearly colorless and clear liquid, or colorless, or clear to slightly opalescent, by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed.

An antibody drug conjugate "retains its physical stability" in a pharmaceutical formulation if it shows no significant increase in aggregation, precipitation and/or denaturation upon visual examination of color and/or clarity, or as measured by UV light scattering, size exclusion chromatography (SEC) and dynamic light scattering (DLS). Changes in protein conformation can be evaluated by fluorescence spectroscopy (which determines the protein tertiary structure) and by FTIR spectroscopy (which determines the protein secondary structure).

An antibody drug conjugate "retains its chemical stability" in a pharmaceutical formulation if it shows no significant chemical change. Chemical stability can be evaluated by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectrometry or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody drug conjugate "retains its biological activity" in a pharmaceutical formulation if the biological activity of the antibody drug conjugate at a given time is within a predetermined range of the biological activity exhibited at the time the pharmaceutical formulation was prepared.

"Optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but does not necessarily, exist.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when amino or hydroxy having free hydrogen is bound to a carbon atom having an unsaturated (e.g., olefinic) bond.

For the preparation of conventional pharmaceutical compositions, refer to Chinese Pharmacopoeia.

The term "carrier" for the drug of the present disclosure refers to a system that can alter how the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

"Giving" and "treating", when applied to animals, humans, experimental subjects, cells, tissues, organs or biological fluids, refer to contact of an exogenous drug, a therapeutic agent, a diagnostic agent or a composition with

the animals, humans, subjects, cells, tissues, organs or biological fluids. "Giving" and "treating" can refer to, for example, therapeutic, pharmacokinetic, diagnostic, research and experimental methods. The treatment of cells comprises contacting the reagent with the cells and contacting the reagent with fluid, where the fluid is in contact with the cells. "Giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or articles of manufacture) may not be effective in alleviating the symptoms of each disease of interest, they shall reduce the symptoms of the disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Effective amount" comprises an amount sufficient to ameliorate or prevent a symptom or condition of a medical disease. An effective amount also refers to an amount sufficient to allow or facilitate diagnosis. The effective amount for a particular patient or veterinary subject may vary depending on the factors such as the condition to be treated, the general health of the patient, the method and route and dosage of administration, and the severity of side effects. An effective amount may be the maximum dose or administration regimen to avoid significant side effects or toxic effects.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis or reconstitution following centrifugation.

## BRIEF DESCRIPTION OF THE DRAWINGS

FIG. 1 shows results of bystander killing activity of ADCs on BxPC3 and MiaPaCa2 mixed cells.

## DETAILED DESCRIPTION

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Preparation Example 1

I. Preparation of Antibodies

The control molecule hRS7 used in the present disclosure is constructed according to patent application WO03074566, and the TINA antibody is constructed according to patent application WO2015098099A1, the sequences of which are as follows:

Heavy chain of hRS7:

*QVQLQQSGSELKKPGASVKVSCKASGYTFTNYGMNWVKQAPGQGLKWMGWINTYTGEPTYTDDFKGRFAFSLDTSVSTAYLQISSLKADDTAVYFCARGGFGSSYWYFDVWGQGSLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK SEQ ID NO: 3;

Light chain of hRS7:

*DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC SEQ ID NO: 4;

Heavy chain of TINA:

*QVQLVQSGAEVKKPGASVKVSCKASGYTFTTAGMQWVRQAPGQGLEWMGWINTHSGVPKYAEDFKGRVTISADTSTSTAYLQLSSLKSEDTAVYYCARSGFGSSYWYFDVWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK SEQ ID NO: 5;

Light chain of TINA:

*DIQMTQSPSSLSASVGDRVTITCKASQDVSTAVAWYQQKPGKAPKLLIYSASYRYTGVP SRFSGSGSGTDFTLTISSLQPEDFAVYYCQQHYITPLTFGQGTKLEIK*RTVAAPSVFIF PPSDEQLKSGTASVVCLLNNFYPREAKVQWKVDNALQSGNSQESVTEQDSKDS TYSLSSTLTLSKADYEKHKVYACEVTHQGLSSPVTKSFNRGEC

SEQ ID NO: 6.

II. Preparation of ADC

<u>Drug loading analysis of ADC stock solution</u>

An ADC is an antibody cross-linked drug, and the mechanism of treating diseases thereof is to transport toxin molecules into cells depending on the targeting performance of the antibody so as to kill the cells. The drug loading plays a decisive role in the drug efficacy.

1. UV-Vis calculation method

The drug loading of the ADC stock solution was determined using the UV method.

Experimental procedures

Cuvettes containing a sodium succinate buffer were separately placed into the reference cell and sample cell, and the absorbance value of the solvent blank was subtracted. Then, a cuvette containing the test sample solution was placed into the sample cell, and the absorbance values at 280 nm and 370 nm were determined.

Calculation for results

The loading capacity of the ADC stock solution was determined by ultraviolet spectrophotometry (instrument: a Thermo nanodrop2000 ultraviolet spectrophotometer), based on the principle that the total absorbance value of the ADC stock solution at a certain wavelength was the sum of the absorbance value of the cytotoxic drug and the monoclonal antibody at the certain wavelength, namely:

$$(1)\ A_{280\ nm} = \varepsilon_{mab\text{-}280} bC_{mab} + \varepsilon_{Drug\text{-}280} bC_{Drug}$$

$\varepsilon_{Drug\text{-}280}$: the mean molar extinction coefficient of the drug at 280 nm of 5100;
$C_{Drug}$: the concentration of the drug;
$\varepsilon_{mab\text{-}280}$: the mean molar extinction coefficient of the monoclonal antibody stock solution at 280 nm of 214,600;
$C_{mab}$: the concentration of the monoclonal antibody stock solution;
b: the optical path length of 1 cm.

Similarly, an equation for the total absorbance value of the sample at 370 nm can be given as:

$$(2)\ A_{370\ nm} = \varepsilon_{mab\text{-}370} bC_{mab} + \varepsilon_{Drug\text{-}370} bC_{Drug}$$

$\varepsilon_{Drug\text{-}370}$: the mean molar extinction coefficient of the drug at 370 nm of 19,000;
$C_{Drug}$: the concentration of the drug;
$\varepsilon_{mab\text{-}370}$: the extinction coefficient of the monoclonal antibody stock solution at 370 nm of 0;
$C_{mab}$: the concentration of the monoclonal antibody stock solution;
b: the optical path length of 1 cm.

The drug loading can be calculated using both equations (1) and (2) as well as the extinction coefficients of the monoclonal antibody and the drug at both wavelengths and their concentrations.

$$\text{Drug loading} = C_{Drug}/C_{mab}.$$

2. CE-SDS calculation method

Reagents and instruments

The adopted was SDS-Mw Analysis Kit produced by Beckman, Cat. # 390953, and containing SDS-MW gel separation buffer, SDS-MW sample buffer, acidic cleaning solution (0.1 mol/L hydrochloric acid solution), basic cleaning solution (0.1mol sodium hydroxide solution) and internal standard substance (10 kDa). The adopted was also the SDS kit produced by Beijing BioCEart Technology Institute, Cat. # BSYK018, and containing CE-SDS gel buffer and CE-SDS sample buffer.

Alkylation solution (0.25 mo iodoacetamide solution): about 0.046 g of iodoacetamide was weighed, 1 mL of ultrapure water was added to dissolve and mix well, and the resulting solution was stored at 2-8 °C for 7 days in dark.

Capillary electrophoresis apparatus: PA800plus from SCIEX.

Capillary: uncoated fused-silica capillary (with an internal diameter of 50 μm), cut to a total length of 30.2 cm and an effective separation length by a high-resolution method of 20 cm.

Preparation of test sample solution

The test sample was diluted to 1 mg/mL with SDS sample buffer. 95 μL of test sample solution (1 mg/mL) was taken and added with 5 μL of iodoacetamide aqueous solution (0.8 mol/L), and the resulting solution was vortexed and mixed well. 95 μL of blank control was taken and added with 5 μL of 0.8 mol/L iodoacetamide aqueous solution, and the resulting solution was vortexed and mixed well. 75 μL of samples were taken from sample tubes and added into the sample vials, and subjected to analysis immediately.

Determination method

1) Pretreatment of capillary: 0.1 mol/L sodium hydroxide solution was washed under 60 psi pressure for 3 min, then washed with 0.1 mol/L hydrochloric acid solution under 60 psi pressure for 2 min, and finally washed with pure water under 70 psi pressure for 1 min. The above pretreatment should be performed before each operation.
2) Pre-filling of capillary: SDS gel separation buffer was washed under 50 psi pressure for 15 min. The above pretreatment should be performed before each operation.

Sample injection: electrokinetic sample injection was performed at 10 kV of reversed polarity, and the reduced sample was injected for 20 s.

Separation: separation was performed at 15 kV of reversed polarity for 40 min.

Temperature of sample chamber: 18 °C to 25 °C.

Temperature of capillary: 18 °C to 25 °C.

Analysis of results

Data were analyzed by using software from Beckman based on the coupling of corresponding drug on sulfydryl liberated from an opened disulfide bond in the antibody, and the proportion of the corrected peak area such as a heavy chain, a non-glycosylated heavy chain and a light chain in the sum of all corrected peak areas was calculated. Calculation formula: DAR = [4 × heavy chain (H) peak area + 2 × half antibody (H-L) peak area + 4 × double heavy chain (H-H) peak area + 2 × heavy-heavy-light chain (H-H-L) peak area]/[heavy chain (H) peak area/2 + half antibody (H-L) peak area/2 + double heavy chain (H-H) peak area + heavy-heavy-light chain (H-H-L) peak area + full antibody peak area], and the weighted average value of the ADC was finally calculated.

III. Reversed-phase high performance liquid chromatography (RP-HPLC)

1. Reagents and instruments:

HPLC system: Waters H-Class ultra-high performance liquid chromatograph UPLC system
Detector: TUV detector (measuring wavelength: 280 nm)
Chromatographic column: BioResolve RP mAb Polyphenyl (2.7 μm 4.6 × 150 mm)

2. Detection conditions:

Column temperature: 80 °C
Flow rate: 1.0 mL/min
Mobile phase A: 0.1% formic acid + 0.025% aqueous trifluoroacetic acid (TFA) solution
Mobile phase B: 0.1% FA + 0.025% trifluoroacetic acid (TFA) in acetonitrile
Gradient program: 27.0% B-45.0% B (0.00 min to 12.00 min), 45.0% B-80.0% B (12.00 min to 13.00 min), 80.0% B-80.0% B (13.00 min to 15.00 min), 27.0% B-27.0% B (15.04 min to 20.00 min)
Sample injection amount: 5.0 μL

3. Data analysis

Compared to an antibody light chain (L0) and an antibody heavy chain (H0) not binding to drugs, the hydrophobicity of the drug-bound light chain (light chain bound to 1 drug: L1) and the drug-bound heavy chains (heavy chain bound to 1 drug: H1, heavy chain bound to 2 drugs: H2, heavy chain bound to 3 drugs: H3, heavy chain bound to 4 drugs: H4) increased in direct proportion to the number of drugs bound thereto, and the retention time was prolonged. Therefore, elution can be performed in the order of L0, L1, H0, H1, H2, H3, and H4. To compare the retention time of L0 and H0, a detection peak was assigned to any one of L0, L1, H0, H1, H2, H3, and H4.

Since the drug linkers absorbed UV, the resulting peak area was corrected according to the number of bound drugs using molar absorption coefficients of the light chains, the heavy chains, and the drug linkers according to the following expression. The calculation formula is as follows:

$$\text{Light chain } (\varepsilon\ \text{LC-280})/(\varepsilon\ \text{LC-280} + \text{number of linked drugs} \times \varepsilon\ \text{drug-280})$$

$$\text{Heavy chain } (\varepsilon\ \text{HC-280})/(\varepsilon\ \text{HC-280} + \text{number of linked drugs} \times \varepsilon\ \text{drug-280})$$

Note: ε LC-280 is molar extinction coefficient of light chain at 280 nm;
ε HC-28 is molar extinction coefficient of heavy chain at 280 nm;
ε drug-280 is molar extinction coefficient of toxin at 280 nm.

Table 1. Calculation table of drug loading from reversed phase chromatography

| Name | Number of linked drugs | Corrected peak area percentage |
|---|---|---|
| $L_0$ | 0 | 100 × $L_0$ corrected peak area/total LC corrected peak area |
| $L_1$ | 1 | 100 × $L_1$ corrected peak area/total LC corrected peak area |
| $H_0$ | 0 | 100 × $H_0$ corrected peak area/total HC corrected peak area |
| $H_1$ | 1 | 100 × $H_1$ corrected peak area/total HC corrected peak area |
| $H_2$ | 2 | 100 × $H_2$ corrected peak area/total HC corrected peak area |
| $H_3$ | 3 | 100 × $H_3$ corrected peak area/total HC corrected peak area |
| $H_4$ | 4 | 100 × $H_4$ corrected peak area/total HC corrected peak area |
| Note: total LC corrected peak area = $L_0$ corrected peak area + $L_1$ corrected peak area. | | |

Total HC corrected peak area = $H_0$ corrected peak area + $H_1$ corrected peak area + $H_2$ corrected peak area + $H_3$ corrected peak area + $H_4$ corrected peak area, and the drug loading of the ADC is calculated as follows:

Drug loading n = 2 × Σ (number of linked drugs × corrected peak area percentage)/100

.

**Preparation Example 2-1 ADC-1**

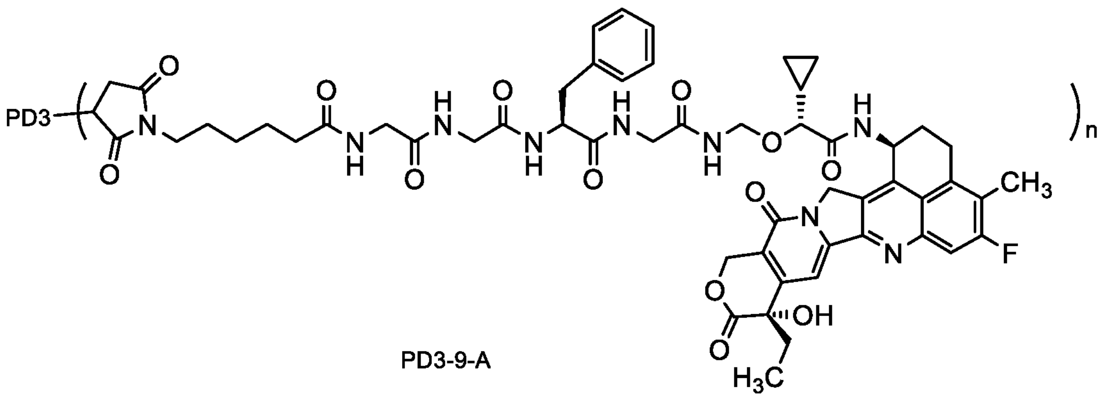

PD3-9-A

To an aqueous PBS buffer of antibody PD3 (0.05 M aqueous PBS buffer at pH 6.5; 10.0 mg/mL, 4.0 mL, 270 nmol) was added at 37 °C a prepared aqueous solution of tris(2-carboxyethyl)phosphine (TCEP) (10 mM, 67.5 μL, 675 nmol). The reaction mixture was shaken on a water bath shaker at 37 °C for 3 h before the reaction was terminated. The reaction mixture was cooled to 25 °C in a water bath.

Compound 9-A (prepared according to 9-A of Example 9 in description of WO2020063676A1, 2.9 mg, 2700 nmol) was dissolved in 180 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give example product ADC-1 of PD3-9-A in PBS buffer (1.93 mg/mL, 18.4 mL), which was then stored at 4 °C.

Average value calculated by UV-Vis: n = 3.77.

**Preparation Example 2-2 ADC-2**

To an aqueous PBS buffer of antibody PD3 (0.05 M aqueous PBS buffer at pH 6.5; 10.0 mg/mL, 4.0 mL, 270 nmol) was added at 37 °C a prepared aqueous solution of tris(2-carboxyethyl)phosphine (TCEP) (10 mM, 143.1 μL, 1431 nmol). The reaction mixture was shaken on a water bath shaker at 37 °C for 3 h before the reaction was terminated. The reaction mixture was cooled to 25 °C in a water bath.

Compound **9-A** (4.35 mg, 4050 nmol) was dissolved in 270 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give example product **ADC-2** of PD3-9-A in PBS buffer (1.69 mg/mL, 17.8 mL), which was then stored at 4 °C.

Average value calculated by UV-Vis: n = 6.59.

**Preparation Example 2-3 ADC-3**

An antibody stock solution containing 145.24 g of PD3 (1.00 mmol, diluted with 10 mM histidine-acetic acid to an antibody concentration of 17.5 mg/mL) and 0.6308 g of tris(2-carboxyethyl)phosphine hydrochloride (Sigma, 2.20 mmol) were stirred in a 10 mM histidine-acetic acid-Tris buffer (pH 7.2) containing 2.5 mM EDTA at 12 °C for 100 min under a constant temperature water bath to give an intermediate I solution. 2 M acetic acid was added dropwise to adjust the pH of the reaction mixture to 5.0. Compound 9-A (4.834 g, 4.50 mmol) was dissolved in 0.456 L of DMSO to give a solution of compound 9-A in DMSO. 0.373 L of DMSO was added to the above intermediate I solution, and the above DMSO solution of compound 9-A was added to the resulting solution, which was then stirred under a water bath at 12 °C for 90 min before the reaction was terminated.

After being filtered by a 0.22 μm filter, the reaction mixture described above was first subjected to buffer exchange to 10% DMSO-30 mM histidine-acetic acid buffer (pH = 5.0) by 15-fold volume equal-volume ultrafiltration (30

kd ultrafiltration membrane) and then subjected to buffer exchange to 30 mM histidine-hydrochloric acid buffer (pH = 6.0) by 18-fold volume equal-volume ultrafiltration, followed by addition of sucrose (to a final concentration of 40 g/L), glycine (to a final concentration of 9 g/L), and tween-80 (to a final concentration of 0.2 g/L), thus obtaining the example product ADC-3 (20 g/L, 132.20 g, yield: 91.0%, average value calculated by reversed phase chromatography: n = 4.0) shown as PD3-9-A; finally lyophilized powder at 80 mg/bottle was prepared.

**Preparation Example 2-4 ADC-4**

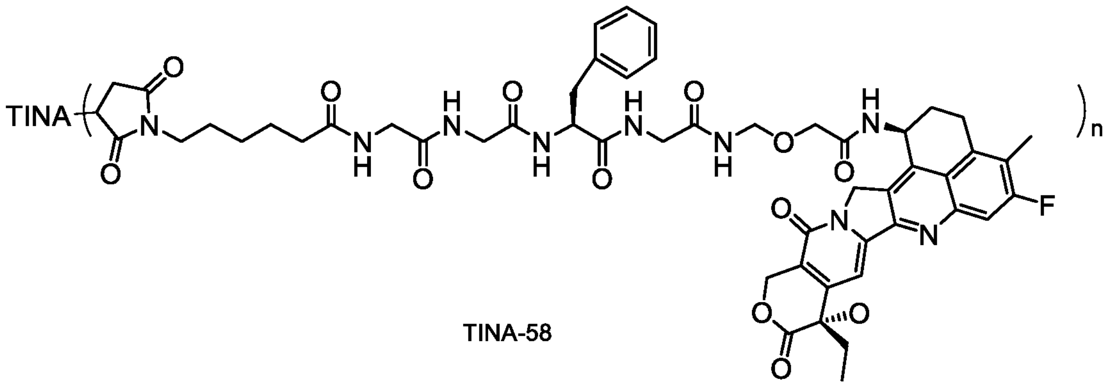

TINA-58

The synthesis was made according to Example 19 in WO2015098099.

To an aqueous PBS buffer of antibody TINA (0.05 M aqueous PBS buffer at pH 6.5; 10.0 mg/mL, 2.0 mL, 135.4 nmol) was added at 37 °C a prepared aqueous solution of tris(2-carboxyethyl)phosphine (TCEP) (10 mM, 33.8 μL, 338 nmol). The reaction mixture was shaken on a water bath shaker at 37 °C for 3 h before the reaction was terminated. The reaction mixture was cooled to 25 °C in a water bath.

Compound 58 (1.4 mg, 1354 nmol) was dissolved in 70 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give the example title product **ADC-4** of TINA-58 in PBS buffer (1.13 mg/mL, 15.1 mL), which was then stored at 4 °C.

Average value calculated by UV-Vis: n = 3.99.

**Preparation Example 2-5 ADC-5**

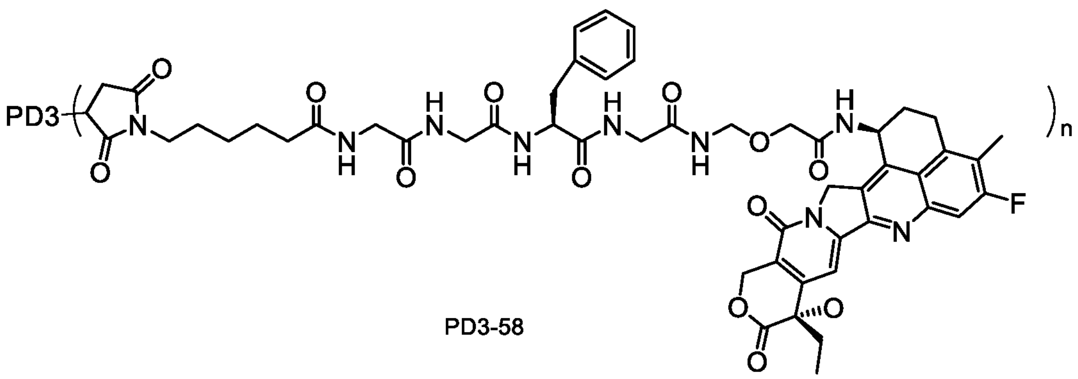

PD3-58

To an aqueous PBS buffer of antibody **PD3** (0.05 M aqueous PBS buffer at pH 6.5; 10.0 mg/mL, 1.5 mL, 101.4 nmol) was added at 37 °C a prepared aqueous solution of tris(2-carboxyethyl)phosphine (TCEP) (10 mM, 25.3 μL, 253 nmol). The reaction mixture was shaken on a water bath shaker at 37 °C for 3 h before the reaction was terminated. The reaction mixture was cooled to 25 °C in a water bath.

Compound 58 (1.05 mg, 1014 nmol, according to Example 58 on page 163 of patent application CN104755494A)

was dissolved in 60 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give the example title product **ADC-5** of PD3-58 in PBS buffer (0.82 mg/mL, 13.5 mL), which was then stored at 4 °C.

Average value calculated by UV-Vis: n = 3.88.

**Preparation Example 2-6 ADC-6**

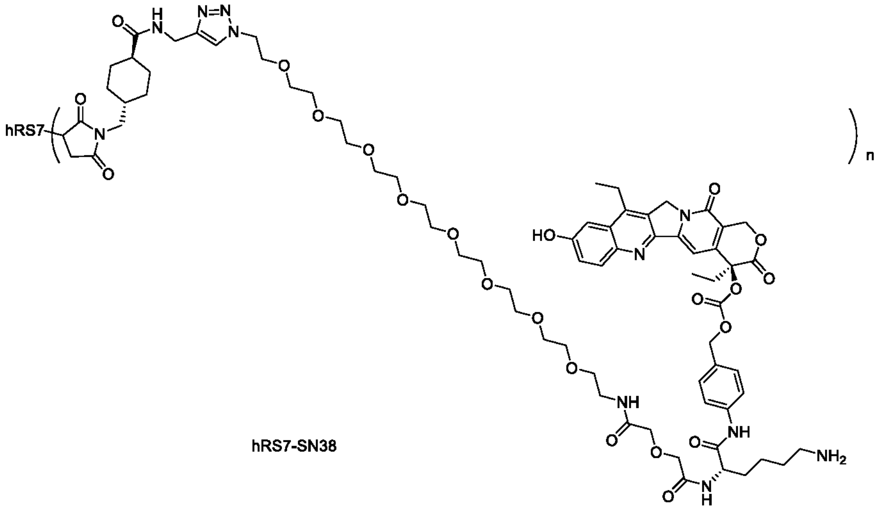

hRS7-SN38

To an aqueous PBS buffer of antibody **hRS7** (0.05 M aqueous PBS buffer at pH 6.5; 10.0 mg/mL, 1.4 mL, 94.60 nmol) was added at 37 °C a prepared aqueous solution of tris(2-carboxyethyl)phosphine (TCEP) (10 mM, 50.1 μL, 501 nmol). The reaction mixture was shaken on a water bath shaker at 37 °C for 3 h before the reaction was terminated. The reaction mixture was cooled to 25 °C in a water bath.

Compound **SN38** (synthesized according to Example 1 on Page 59 of patent application CN105407891A, 2.1 mg, 1419 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give the example title product **ADC-6** of **hRS7**-SN38 in PBS buffer (1.03 mg/mL, 11.5 mL), which was then stored at 4 °C.

Average value calculated by CE-SDS: n = 7.56.

**Preparation Example 2-7 ADC-7**

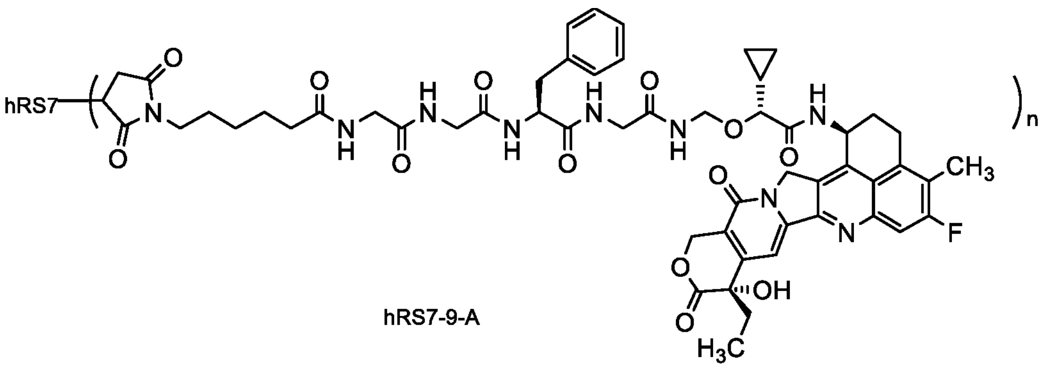

hRS7-9-A

To an aqueous PBS buffer of antibody **hRS7** (0.05 M aqueous PBS buffer at pH 6.5; 10.0 mg/mL, 2.18 mL, 147.3 nmol) was added at 37 °C a prepared aqueous solution of tris(2-carboxyethyl)phosphine (TCEP) (10 mM, 36.8

μL, 368 nmol). The reaction mixture was shaken on a water bath shaker at 37 °C for 3 h before the reaction was terminated. The reaction mixture was cooled to 25 °C in a water bath.

Compound **9-A** (1.58 mg, 1471 nmol) was dissolved in 100 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give the example title product **ADC-7** of **hRS7**-9-A in PBS buffer (1.10 mg/mL, 16.4 mL), which was then stored at 4 °C.

Average value calculated by UV-Vis: n = 3.72.

**Preparation Example 2-8 ADC-8**

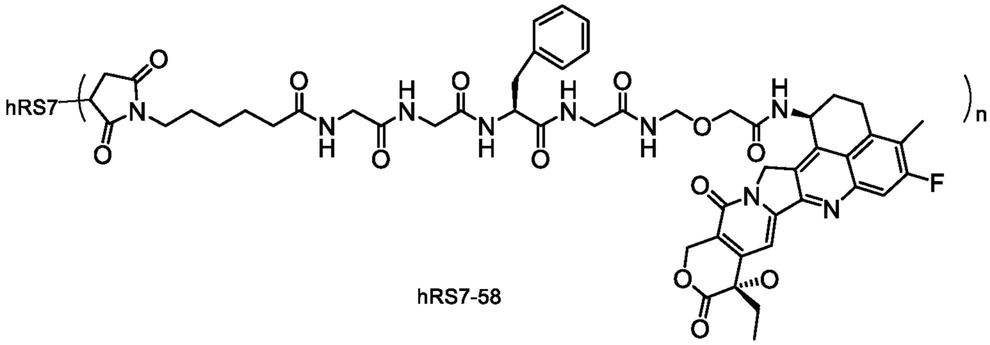

hRS7-58

To an aqueous PBS buffer of antibody **hRS7** (0.05 M aqueous PBS buffer at pH 6.5; 10.0 mg/mL, 2.18 mL, 147.3 nmol) was added at 37 °C a prepared aqueous solution of tris(2-carboxyethyl)phosphine (TCEP) (10 mM, 36.8 μL, 368 nmol). The reaction mixture was shaken on a water bath shaker at 37 °C for 3 h before the reaction was terminated. The reaction mixture was cooled to 25 °C in a water bath.

Compound 58 (1.52 mg, 1473 nmol, according to Example 58 on page 163 of patent application CN104755494A) was dissolved in 100 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give the example title product **ADC-8** of **hRS7**-58 in PBS buffer (1.02 mg/mL, 16.8 mL), which was then stored at 4 °C.

Average value calculated by UV-Vis: n = 3.93.

**Preparation Example 2-9 ADC-9**

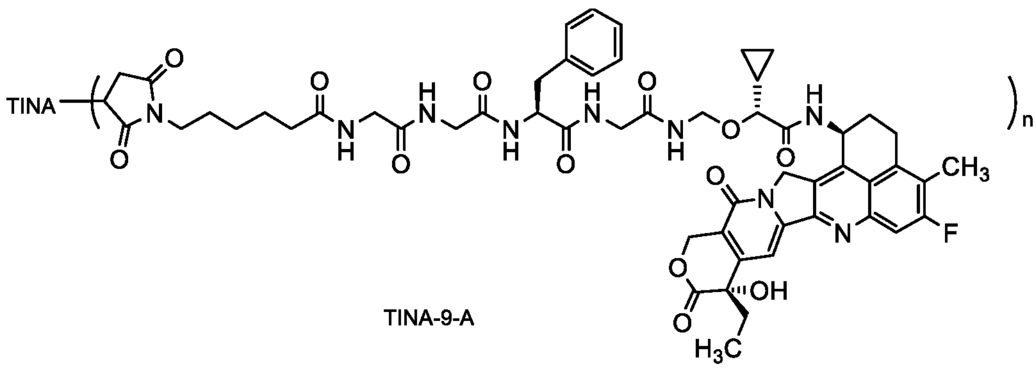

TINA-9-A

To an aqueous PBS buffer of antibody **TINA** (0.05 M aqueous PBS buffer at pH 6.5; 10.0 mg/mL, 0.95 mL, 64.2 nmol) was added at 37 °C a prepared aqueous solution of tris(2-carboxyethyl)phosphine (TCEP) (10 mM, 16.0 μL, 160 nmol). The reaction mixture was shaken on a water bath shaker at 37 °C for 3 h before the reaction was terminated. The reaction mixture was cooled to 25 °C in a water bath.

Compound **9-A** (0.69 mg, 642 nmol) was dissolved in 30 μL of DMSO, and the resulting solution was added

to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give the example title product **ADC-9** of TINA-9-A in PBS buffer (0.99 mg/mL, 7.0 mL), which was then stored at 4 °C.

Average value calculated by UV-Vis: n = 3.99.

The activity of the antibodies and conjugates of the present disclosure is verified with biochemical test methods.

**Test Example 1: Antibody Protein Level Binding Assay**

hTROP-2 protein was diluted to 1 μg/mL with PBS buffer at pH 7.4 (Shanghai BasalMedia Technologies Co., LTD., B320), added into a 96-well microplate at 100 μL per well, and incubated at 4 °C overnight. After the liquid was discarded, 300 μL of 5% skim milk (BD, 232100) diluted with PBS was added to each well for blocking, and the mixture was incubated at 37 °C for 2 h. After the blocking was completed, the blocking solution was discarded, and the plate was washed 3 times with a PBST buffer (pH 7.4, PBS containing 0.1% tween-20). 100 μL of the antibody solution diluted in a gradient was added to each well, and the mixture was incubated at 37 °C for 1 h. After the incubation was completed, the plate was washed 3 times with PBST. 100 μL of mouse anti-human IgG (H+L) (Jackson ImmunoResearch, 209-035-088, 1:8000 dilution) was added to each well, and the mixture was incubated at 37 °C for 1 h. After the plate was washed 3 times with PBST buffer, 100 μL of TMB chromogenic substrate (KPL, 5120-0077) was added into each well and incubated at room temperature for 10-15 min, and the reaction was terminated by adding 50 μL of 1 M $H_2SO_4$ into each well. The plate was detected for the absorbance value at 450 nm using a microplate reader, the binding curves of antibodies to antigen were fitted with software, and the $EC_{50}$ value was calculated. The binding activity of the antibodies to the proteins is shown in Table 2.

Table 2. Antibody protein level binding activity

| Antibody | PD3 | hRS7 | TINA |
|---|---|---|---|
| Emax (OD value) | 1.40 | 1.37 | 1.39 |
| $EC_{50}$ (nM) | 13.6 | 18.29 | 16.14 |

The result shows that the PD3 antibody in the present application has higher binding activity to hTROP-2 protein.

**Test Example 2: Antibody Cell Level Binding Assay**

The stably transfected TROP-2-expressing CHOK1 cells were suspended in FACS buffer (2% fetal bovine serum (Gibco, 10099141) pH 7.4 PBS (Sigma, P4417-100TAB)) to give a 1 × $10^6$/mL cell suspension, which was then added to a 96-well round-bottom plate at 100 μL/well. After centrifugation and removal of the supernatant, the test antibodies diluted with FACS buffer to different concentrations were added at 50 μL/well. The plate was incubated in the dark in a 4 °C refrigerator for 1 h. The plate was washed 3 times with FACS buffer by centrifugation at 300 g, and Alexa Fluor 488 goat anti-human IgG (H+L) (invitrogen, A-11013) at working concentration was added. The plate was incubated in the dark in a 4 °C refrigerator for 40 min. The plate was washed 3 times with FACS buffer by centrifugation at 300 g and tested on a BD FACSCantoII flow cytometer for geometric mean fluorescence intensity (MFI). The binding $EC_{50}$ value of the antibody to the stably transfected TROP-2-expressing cells was calculated. The binding activity of the antibodies to the cells is shown in Table 3.

Table 3. Antibody cell-level binding activity

| Antibody | PD3 | hRS7 | TINA |
|---|---|---|---|
| Emax (MFI) | 16693 | 16050 | 14217 |
| $EC_{50}$ (nM) | 1.57 | 2.31 | 3.55 |

The result shows that the PD3 antibody in the present application has higher binding activity to TROP-2 protein-expressing cells.

**Test Example 3: Antibody Endocytosis Assay**

The purpose of the assay is that the activated DT kills cells after the DT3C protein enters the cells, indirectly

reflecting endocytosis of the anti-TROP-2 antibody. The *in vitro* endocytic activity of the antibody is evaluated according to $EC_{50}$ and Emax.

DT3C is a recombinantly expressed fusion protein and is formed by fusing fragment A of diphtheria toxin (toxin part only) and fragment 3C of group G streptococcus (IgG binding part). The protein can have a high affinity for IgG part of antibody, enter cells together with the IgG part when the antibody is endocytosed, and release toxic DT under the action of intracellular furin protease. The DT can inhibit the activity of EF2-ADP ribosylation, block the protein translation process and finally cause cell death. DT3C that does not enter the cell has no activity of cell killing. The endocytic activity of the antibody was evaluated according to cell killing.

Cell suspensions were prepared with fresh cell medium containing 20% low IgG FBS at a cell density of $2 \times 10^4$ cells/mL, and added into the cell culture plates at 50 μL/well and incubated with 5% carbon dioxide at 37 °C for 16 h. DT3C at 4× concentration was formulated in serum-free medium and filtered through a 0.22 μm filter to obtain a sterile solution. Antibody at 4× concentration was prepared in serum-free medium, and 80 μL of DT3C and 80 μL of antibody were mixed at a volume of 1:1, and incubated at room temperature for 30 min. 50 μL of the diluted antibody-DT3C mixture was added to 50 μL of cells and incubated in an incubator for three days. 50 μL of CTG was added into each well and incubated for 10 min at room temperature in dark, and the chemiluminescence values were detected using Victor3. The endocytic activity of the antibody is shown in Table 4.

Table 4. Antibody endocytic activity

| Antibody | PD3 | hRS7 | TINA |
|---|---|---|---|
| Emax | 99.1% | 98.5% | 98.7% |
| EC50 (nM) | 0.15 | 0.18 | 0.18 |

The results show that the PD3 antibody in the present application has higher endocytosis efficiency.

**Test Example 4: Affinity Assay of Antibody**

The affinity of antibody for TROP-2 was detected in a form of a capture antibody. The antibody was affinity-captured by a Protein A (Cat. # 29127556, GE) biosensor chip conjugated with an anti-human IgG antibody (Cat. # BR-1008-39, Lot. # 10260416, GE), then an antigen hTROP-2 flowed on the surface of the chip, and a Biacore T200 instrument was used for detecting reaction signals in real time to obtain association and dissociation curves. After dissociation was completed for each assay cycle, the chip was washed clean and regenerated with regeneration buffer, Glycine1.5 (Cat. # BR100354, GE) or 3 M $MgCl_2$ (from Human antibody capture kit, Cat. # BR100839, GE). After the assay was completed, the data were fitted with (1:1) Langmuir model using GE Biacore T200 Evaluation version 3.0 to obtain affinity values. The affinity of the antibody for the proteins is shown in Table 5.

Table 5. Affinity of antibody determined by Biacore

| Antibody | PD3 | hRS7 | TINA |
|---|---|---|---|
| KD (M) | 6.86E-10 | 9.87E-10 | 2.15E-8 |

The results show that the PD3 antibody in the present application has a higher affinity for hTROP-2.

**Test Example 5: Cell Activity of ADC Molecules**

The cells used in this assay were as follows: FaDu(+++) purchased from ATCC, Cat. # HTB-43™; HCC827(+++), purchased from ATCC, Cat. # CRL-2868; Colo205(++), purchased from Cell Bank, Chinese Academy of Sciences, Cat. # TCHu102; DMS53(++), purchased from ATCC, Cat. # CRL-2062™; SK-OV-3(+), purchased from ATCC, Cat. # HTB-77; CHO-K1(-), purchased from ATCC, Cat. # CCL-61™; wherein "+" represents the expression amount of TROP-2 in the cell population, more "+" means that the expression amount of TROP-2 is higher, and "-" means that TROP-2 is not expressed.

Cell suspensions were prepared with fresh cell medium containing 10% FBS at a density of 3703 cells/mL, and added into the 96-well cell culture plate at 135 μL/well and incubated with 5% carbon dioxide at 37 °C for 16 h. ADC samples were formulated to be 5 μM with PBS. This concentration was taken as the initial concentration, and the sample was 5-fold diluted with PBS to a total of 8 concentrations. 15 μL of the above ADC solution was added into each well. The plate was cultured at 37 °C in 5% carbon dioxide for 6 days. 70 μL of CTG was added into each well and incubated

for 10 min at room temperature in dark, the chemiluminescence values were detected using Victor3, and data analysis was performed using the GraphPad Prism software.

The result shows that ADC-1 has stronger cell killing effect, and the killing effect is positively correlated with the TROP-2 expression level on the surface of the tumor cell.

Table 6. Killing activity of ADC on cells with different TROP-2 expression levels

| | FaDu | | HCC827 | | Colo205 | | DMS53 | | SK-OV-3 | | CHO-K1 | |
|---|---|---|---|---|---|---|---|---|---|---|---|---|
| Sample | $EC_{50}$ (nM) | Emax (%) | $EC_{50}$ (nM) | Emax (%) | $EC_{50}$ (nM) | Emax (%) | $EC_{50}$ (nM) | Emax (%) | $EC_{50}$ (nM) | Emax (%) | $EC_{50}$ (nM) | Emax (%) |
| ADC-1 | 0.23 | 100.42 | 0.14 | 89.64 | 39.23 | 99.38 | 42.46 | 89.39 | NA | 61.91 | NA | 9.31 |
| ADC-4 | 0.53 | 101.25 | 0.27 | 88.16 | 59.8 | 99.66 | 75.58 | 89.83 | NA | 49.15 | NA | 1.71 |
| ADC-7 | 0.31 | 100.75 | 0.18 | 89.33 | 52.75 | 99.52 | 72.92 | 88.74 | NA | 66.11 | NA | 8.69 |

A second parallel comparison assay was performed in the method as described above, and the obtained results are as follows:

Table 7. Killing activity of ADC on cells with different TROP-2 expression levels

| | FaDu | | HCC827 | | Colo205 | | DMS53 | |
|---|---|---|---|---|---|---|---|---|
| **Sample** | $EC_{50}$ (nM) | $E_{max}$ (%) | $EC_{50}$ (nM) | $E_{max}$ (%) | $EC_{50}$ (nM) | $E_{max}$ (%) | $EC_{50}$ (nM) | $E_{max}$ (%) |
| ADC-1 | 0.08 | 101.6 | 0.06 | 89.57 | 12.07 | 100.34 | 15.08 | 93.24 |
| ADC-5 | 0.14 | 101.58 | 0.13 | 88.23 | 25.63 | 99.64 | 25.71 | 92.47 |

**Test Example 6: Bystander Killing Activity Study**

BxPC3 (human pancreatic cancer cells, ATCC, CRL-1687) and MiaPaCa2 cells (human pancreatic cancer cells, biocytogen, B-HCL-014) were cultured with RPMI1640+10% FBS and DMEM/high glucose+10% FBS, respectively; cells were trypsinized, neutralized with fresh medium, and centrifuged at 1000 rpm for 3 min; supernatant was discarded, and cells were resuspended with RPMI1640+10% FBS. After the cells were counted, the cell density of BxPC3 was adjusted to $6 \times 10^4$ cells/mL and the cell density of MiaPaCa2-luc was adjusted to $1.5 \times 10^4$ cells/mL. 500 $\mu$L of BxPC3 cells and 500 $\mu$L of MiaPaCa2-luc cells were added into each well of a 12-well plate 1. 500 $\mu$L of MiaPaCa2-luc cells and 500 $\mu$L of RPMI1640 medium containing 10% FBS serum were added into a 12-well plate 2. The plates were cultured at 37 °C in 5% carbon dioxide for 24 h.

The ADC samples were formulated into 40$\times$ concentration of intermediate solutions (0.2 $\mu$M). 25 $\mu$L of the above samples were taken and added into the corresponding well of the 12-well plates. Solvent control group was set. The plates were cultured at 37 °C in 5% carbon dioxide for 6 days. The cells in the 12-well plates were trypsinized, neutralized with fresh medium, and centrifuged at 1000 rpm for 3 min. The supernatant was discarded, and the cells were resuspended in 1 mL of FACS buffer (PBS + 2.5% FBS).

20 $\mu$L of the cells were taken and stained with 20 $\mu$L of trypan blue and counted. The cells in the plate 1 were centrifuged at 1000 rpm for 3 min, the supernatant was discarded, the cells were resuspended in 100 $\mu$L of FACS Buffer, 2 $\mu$L of TROP-2 (EGP-1) monoclonal antibody (MR54) was added, and the cells were incubated on ice for 30 min. The cells were centrifuged at 2000 rpm at 4 °C for 1 min, the supernatant was discarded, and the cells were resuspended in 150 $\mu$L of FACS buffer. Detection was performed using BD FACSVerse. Data was analyzed by Flowjo 7.6. The results of the bystander killing activity study are shown in FIG. 1.

The results show that ADC-1 in the present disclosure has a clear bystander killing effect, and ADCs do not kill the TROP-2 negative MiaPaCa2 cells, but ADC-1 also kills TROP-2 negative cells when the TROP-2 expressing BxPC3 cells are mixed with the negative cells MiaPaCa2.

Biological Evaluation ***of In Vivo* Activity**

**Test Example 7: *In Vivo* Efficacy Evaluation of Fadu Cell CDX Mouse Model**

Fadu cells ($3 \times 10^6$) were subcutaneously inoculated in the right flank of Balb/c nude mice, after 10 days of

inoculation, the nude mice with heavy weight, too big tumor and too small tumor were removed after the tumor volume was about 245 $mm^3$, and the remaining mice were randomized into 5 groups of 8 mice according to tumor volume. Each mouse was intraperitoneally injected with an ADC at 0.1 mL/10 g body weight at days 0 and 8, making a total of 2 injections with a dose of 1 mg/kg. The tumor volumes and body weights were measured twice a week and the results were recorded for 21 days.

Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA or One-way ANOVA.

Tumor volume (V) was calculated as: $V = 1/2 \times L_{long} \times L_{short}^2$

Relative tumor proliferation rate $T/C(\%) = (T - T_0)/(C - C_0) \times 100$, wherein T and C are the tumor volumes of animals at the end of the experiment in the treatment group and the control group, respectively; $T_0$ and $C_0$ are the tumor volumes of animals at the beginning of the experiment in the treatment group and the control group, respectively.

Tumor growth inhibition TGI (%) = 1 - T/C (%).

The results are shown in Table 8, which indicates that ADC-1 has a strong tumor-inhibiting effect on FaDu xenograft tumors at a dose of 1 mpk.

Table 8. Efficacy of ADC on FaDu xenograft tumors of tumor-bearing nude mice

| **Group** | **Mean tumor volume ($mm^3$)** | | **Mean tumor volume ($mm^3$)** | | **Tumor inhibition rate TGI (%)** |
|---|---|---|---|---|---|
| | **D0** | **SEM** | **D21** | **SEM** | |
| Blank control | 246.8 | 29.04 | 2245.04 | 275.14 | N/A |
| ADC-4 1mg/kg | 244.41 | 29.62 | 1180.96 | 193.55 | 53 |
| ADC-1 1mg/kg | 251.19 | 23.45 | 238.54 | 107.62 | 101 |
| ADC-2 1mg/kg | 270.48 | 21.36 | 0 | 0 | 114 |
| PD3 30mg/kg | 246.02 | 25 | 2407.37 | 207.71 | -8 |

### Test Example 8: *In Vivo* Efficacy Evaluation of SKOV3 Cell CDX Mouse Model

SKOV3 cells ($5 \times 10^6$) were subcutaneously inoculated in the right flank of Balb/c nude mice, after 23 days of inoculation, the nude mice with heavy weight, too big tumor and too small tumor were removed after the tumor volume was about 180 $mm^3$, and the remaining mice were randomized into 5 groups of 8 mice according to tumor volume. Each mouse was intraperitoneally injected with an ADC at 0.1 mL/10 g body weight for a total of 2 injections, and the dose is shown in following table. The tumor volumes and body weights were measured twice a week and the results were recorded. Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA or One-way ANOVA.

Tumor volume (V) was calculated as: $V = 1/2 \times L_{long} \times L_{short}^2$

Relative tumor proliferation rate $T/C(\%) = (T - T_0)/(C - C_0) \times 100$, wherein T and C are the tumor volumes of animals at the end of the experiment in the treatment group and the control group, respectively; $T_0$ and $C_0$ are the tumor volumes of animals at the beginning of the experiment in the treatment group and the control group, respectively.

Tumor growth inhibition TGI (%) = 1 - T/C (%).

The results are shown in Table 9, which indicates that ADC-1 has a strong tumor-inhibiting effect on SKOV3 xenograft tumors at different doses, and the tumor-inhibiting effect is dose-dependent.

The effect is dose-dependent.

Table 9. Efficacy of ADC on SKOV3 xenograft tumors of tumor-bearing nude mice

| **Group** | **Mean tumor volume ($mm^3$)** | | **Mean tumor volume ($mm^3$)** | | **Tumor inhibition rate TGI (%)** |
|---|---|---|---|---|---|
| | D0 | SEM | D21 | SEM | |
| Blank control | 183.26 | 7.6 | 1081.36 | 132.88 | N/A |
| ADC-110mg/kg | 185.48 | 9.62 | 302.93 | 39.34 | 87 |

(continued)

| Group | Mean tumor volume ($mm^3$) | | Mean tumor volume ($mm^3$) | | Tumor inhibition rate TGI (%) |
|---|---|---|---|---|---|
| | D0 | SEM | D21 | SEM | |
| ADC-1 3mg/kg | 185.09 | 10.07 | 519.9 | 38.93 | 63 |
| ADC-1 1mg/kg | 183.29 | 8.87 | 795.34 | 124.81 | 32 |
| ADC-4 10mg/kg | 183.83 | 8.9 | 938.99 | 148.98 | 16 |
| ADC-4 3mg/kg | 184.17 | 8.2 | 873.38 | 50.84 | 23 |
| ADC-4 1mg/kg | 185.17 | 8.38 | 1110.52 | 159.73 | -3 |
| PD3 30mg/kg | 182.85 | 8.38 | 1384.28 | 93.87 | -34 |

**Test Example 9: *In Vivo* Efficacy Evaluation of Colo205 Cell CDX Mouse Model**

Colo205 cells ($5 \times 10^6$) were subcutaneously inoculated in the right flank of Balb/c nude mice, after 10 days of inoculation, the nude mice with heavy weight, too big tumor and too small tumor were removed after the tumor volume was about 245 $mm^3$, and the remaining mice were randomized into 6 groups of 8 mice according to tumor volume. Each mouse was intraperitoneally injected with an ADC at 0.1 mL/10 g body weight at day 0 (D0) and day 10, making a total of 2 injections with a dose of 10 mg/kg. The tumor volumes and body weights were measured twice a week and the results were recorded for 28 days (D28).

Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA or One-way ANOVA.

Tumor volume (V) was calculated as: $V = 1/2 \times L_{long} \times L_{short}^2$

Relative tumor proliferation rate T/C(%) = $(T - T_0)/(C - C_0) \times 100$, wherein T and C are the tumor volumes of animals at the end of the experiment in the treatment group and the control group, respectively; $T_0$ and $C_0$ are the tumor volumes of animals at the beginning of the experiment in the treatment group and the control group, respectively. Tumor growth inhibition TGI (%) = 1 - T/C (%).

The results are shown in Table 10, which indicates that ADC-9 and ADC-7 prepared by conjugation to compound 9-A have a strong tumor-inhibiting effect on Colo205 xenograft tumors at a dose of 1 mpk.

Table 10. Efficacy of ADC on FaDu xenograft tumors of tumor-bearing nude mice

| Group | Mean tumor volume ($mm^3$) | | Mean tumor volume ($mm^3$) | | Tumor inhibition rate TGI (%) |
|---|---|---|---|---|---|
| | D0 | SEM | D28 | SEM | |
| Blank control | 210.30 | 24.91 | 1355.56 | 117.46 | N/A |
| ADC-6 10mg/kg | 194.93 | 26.56 | 1344.84 | 95.93 | 0 |
| ADC-7 10mg/kg | 194.64 | 21.03 | 35.13 | 12.80 | **113** |
| ADC-8 10mg/kg | 199.09 | 23.41 | 475.66 | 175.24 | 76 |
| ADC-9 10mg/kg | 206.70 | 21.21 | 99.86 | 32.55 | **109** |
| ADC-4 10mg/kg | 201.52 | 27.85 | 513.83 | 118.53 | 73 |

**Formulation**

**The equipment used in the formulation preparation and determination and the calculation method for results are shown below:**

**1) SEC molecular exclusion chromatography:**

This is a method for analyzing the separation of a solute by the relative relationship between the pore size of the gel pores and the size of the polymer sample molecule coil.

SEC% (SEC monomer content percentage) = A monomer/A total × 100% (A monomer represents the peak area of the main peak monomer in the sample, and A total represents the sum of all peak areas).

Instrument for SEC measurement: Agilent 1260; column: waters, XBrige BEH200Å SEC (300 × 7.8 mm 3.5 μm)

**2) R-CE capillary gel electrophoresis:**

This is a method of moving the gel into a capillary as a supporting medium for electrophoresis and performing separation according to the molecular weight of the sample under a certain voltage.

R-CE purity percentage = A main peak/A total × 100% (A main peak represents the light chain main peak area + the heavy chain main peak area in the sample, and A total represents the sum of all peak areas).

Instrument for R-CE determination: Beckman model plus800

**3) Turbidity measurement:**

The degree to which light is hindered from passing through a water layer indicates the ability of the water layer to scatter and absorb light. It is related not only to the suspended matter content but also to the particle composition, size and shape as well as the reflection performance of its surface. In comparing the absorption values of the same protein sample at the same concentration at the same wavelength (within near ultraviolet and visible wavelength regions), the greater the absorption value is, the greater the turbidity is, the more the protein molecules in the sample tend to aggregate. The instrument for measurement was a multifunctional microplate reader (Molecular Devices M5). Samples in the same volume were added to a 96-well plate and the absorbance values were read.

**4) Osmotic pressure determination:**

The freezing point method is used for determining the osmotic pressure. The freezing point of a solution is determined by using a high-sensitivity temperature-sensing element on the basis of the proportional relation between the freezing point depression value and the molar concentration of the solution, and then converted into the osmotic pressure through electric quantity. Manufacturer of instrument: Loser, model: OM815.

**5) Protein concentration determination:**

In the present disclosure, the concentration of the antibody drug conjugate is expressed in protein concentration, i.e., the concentration of the antibody moiety in the antibody drug conjugate.

Since the toxin in the antibody drug conjugate absorbs light at a protein characteristic absorption wavelength of 280 nm and also at 370 nm, the protein concentration is calculated by the following formula:

$$A_{280nm} = (C_{drug} \times E_{drug-280} + C_{mAb} \times E_{mAb-280}) \times l$$

$$A_{370nm} = C_{drug} \times E_{drug-370} \times l$$

$$\text{if} \quad R = \frac{E_{drug-370}}{E_{drug-280}}$$

that is:

$$C_{mAb} = \frac{R \times A_{280nm} - A_{370nm}}{R \times E_{mAb-280}}$$

In the formula, $A_{280\,nm}$: the average value of the absorbance of a single piece of the test sample solution at the wavelength of 280 nm when the optical path is 1 cm;

$A_{370\ nm}$: the average value of the absorbance of a single piece of the test sample solution at the wavelength of 370 nm when the optical path is 1 cm;
$E_{mAb-280}$: the mass extinction coefficient of the protein at the wavelength of 280 nm, being 1.532 $g^{-1}cm^{-1}L$;
$E_{dug-280}$: the mass extinction coefficient of the toxin at the wavelength of 280 nm, being 5.17 $g^{-1}cm^{-1}L$;
$E_{drug-370}$: the mass extinction coefficient of the toxin at the wavelength of 370 nm, being 17.89 $g^{-1}cm^{-1}L$;
R: the ratio of the extinction coefficient of the toxin at 370 nm to that at 280 nm, being 3.46;
$C_{mAb}$: protein concentration, mg/mL;
1: optical path length, cm (here the optical path is 1 cm).

If the test sample solution is diluted, the protein concentration is as follows: C (mg/ mL) = $C_{mAb} \times N$, and N is a dilution factor.

Instrument for protein concentration determination: ultraviolet-visible spectrophotometer, model: Nano Drop One.

**Example 1: Screening of Buffer System and pH for Anti-Trop2-ADC Antibody Formulations**

The following buffers were used to prepare anti-Trop2-ADC antibody formulations with the protein concentration of ADC-3 being 10-30 mg/mL. Specific formula composition is as follows:

1) 10 mM citric acid-sodium citrate (CA for short), pH 5.0, at a protein concentration of 20 mg/mL;
2) 10 mM CA, pH 5.5, at a protein concentration of 20 mg/mL;
3) 10 mM CA, pH 6.0, at a protein concentration of 20 mg/mL;
4) 10 mM CA, pH 6.5, at a protein concentration of 20 mg/mL;
5) 10 mM succinic acid-sodium succinate (SA for short), pH 5.0, at a protein concentration of 20 mg/mL;
6) 10 mM SA, pH 5.5, at a protein concentration of 20 mg/mL;
7) 10 mM SA, pH 6.0, at a protein concentration of 20 mg/mL;
8) 10 mM histidine-histidine hydrochloride (His-HCl for short), pH 5.5, at a protein concentration of 10 mg/mL;
9) 10 mM His-HCl, pH 5.5, at a protein concentration of 20 mg/mL;
10) 10 mM His-HCl, pH 5.5, at a protein concentration of 30 mg/mL;
11) 10 mM His-HCl, pH 6.0, at a protein concentration of 20 mg/mL;
12) 10 mM His-HCl, pH 6.5, at a protein concentration of 20 mg/mL;

The prepared formulations were filtered sterilely and placed in containers, which were plugged and capped. The stability of the samples under a forced degradation condition (40 °C high temperature) was examined, and the appearance, SEC, and R-CE were used as evaluation indexes to examine the stability of the formulations. The experimental results are shown in Table 11.

At 40 °C and M1, the particle number of the His-HCl system was less than those of the CA and SA systems according to appearance inspection, the purity of the His system was higher than those of the CA and SA systems at the same pH value and the same protein concentration, and the SEC monomer peak and the R-CE main peak decrease value became smaller along with the increase of the pH value in the same buffer system. In the 10 mM pH5.5 His system, there was a slight increase in SEC polymers along with increasing protein concentration, and there was no significant change in the R-CE purity. In summary, the buffer system is preferably His-HCl, the pH range is preferably 5.5-6.5, and the protein concentration is preferably 10 mg/mL to 30 mg/mL.

Table 11. Experimental results of screening of pH and buffer systems

| No. | Storage conditions | Appearance | SEC% | | | R-CE % | |
|---|---|---|---|---|---|---|---|
| | | | Polymer | Monomer | △monomer | Main peak | △main peak |
| 1 | DO | Clear and transparent | 0.5 | 98.9 | | 99.2 | |
| | 40 °CM1 | Slightly opalescent, with a large number of particles | 2.1 | 87.1 | 11.8 | 88.8 | 10.5 |
| 2 | DO | Clear and transparent | 0.6 | 98.8 | | 99.2 | |
| | 40 °CM1 | Slightly opalescent, with a large number of particles | 3.4 | 90.4 | 8.4 | 92.9 | 6.4 |

(continued)

| No. | Storage conditions | Appearance | SEC% | | | R-CE % | |
|---|---|---|---|---|---|---|---|
| | | | Polymer | Monomer | △monomer | Main peak | △main peak |
| 3 | DO | Clear and transparent | 0.6 | 98.8 | | 99.3 | |
| | 40 °CM1 | Slightly opalescent, with a large number of particles | 3.8 | 92.7 | 6.2 | 95.5 | 3.8 |
| 4 | DO | Clear and transparent | 0.7 | 98.7 | | 99.2 | |
| | 40 °CM1 | Slightly opalescent, with a large number of particles | 3.7 | 93.6 | 5.1 | 95.9 | 3.3 |
| 5 | DO | Clear and transparent | 0.5 | 99.0 | | 99.1 | |
| | 40 °CM1 | Slightly opalescent, with a large number of particles | 1.6 | 91.0 | 8.0 | 91.5 | 7.5 |
| 6 | DO | Clear and transparent | 0.6 | 98.8 | | 99.0 | |
| | 40 °CM1 | Slightly opalescent, with a large number of particles | 2.8 | 92.5 | 6.3 | 93.9 | 5.1 |
| 7 | DO | Clear and transparent | 0.8 | 98.6 | | 99.2 | |
| | 40 °CM1 | Slightly opalescent, with a large number of particles | 3.9 | 93.2 | 5.4 | 95.7 | 3.6 |
| 8 | DO | Clear and transparent | 0.5 | 99.0 | | 99.4 | |
| | 40 °CM1 | Slightly opalescent, with particles | 0.7 | 95.4 | 3.6 | 95.9 | 3.5 |
| 9 | DO | Clear and transparent | 0.5 | 99.5 | | 99.2 | |
| | 40 °CM1 | Slightly opalescent, with particles | 1.0 | 94.4 | 5.1 | 94.9 | 4.4 |
| 10 | DO | Clear and transparent | 0.5 | 98.9 | | 98.9 | |
| | 40 °CM1 | Slightly opalescent, with particles | 1.4 | 93.9 | 5.0 | 95.5 | 3.4 |
| 11 | DO | Clear and transparent | 0.5 | 98.9 | | 99.2 | |
| | 40 °CM1 | Slightly opalescent, with particles | 1.4 | 95.2 | 3.6 | 96.0 | 3.2 |
| 12 | DO | Clear and transparent | 0.6 | 98.8 | | 99.0 | |
| | 40 °CM1 | Slightly opalescent, with particles | 2.0 | 95.3 | 3.6 | 95.8 | 3.3 |
| Note: D0 indicates the start of the experiment, "M" indicates month, e.g. M1 indicates one month; the △ value indicates the difference between the test item and D0 after storage under each condition, and the same applies below. | | | | | | | |

**Example 2: Screening of Surfactants for Anti-Trop2-ADC Antibody Formulations**

Anti-Trop2-ADC antibody formulations containing 80 mg/mL sucrose and polysorbate 80 (PS80 for short) at different concentrations with the protein concentration of ADC-3 being 20 mg/mL were prepared by using a 10 mM pH 6.0 His-HCl buffer system. Specific formula composition is as follows:

1) Free of PS80;
2) 0.2 mg/mL PS80;
3) 0.4 mg/mL PS80;
4) 0.6 mg/mL PS80;

The prepared formulations were filtered sterilely and placed in containers, which were plugged and capped. The samples were subjected to shaking (25 °C, 300 rpm, 3 days) and high temperature (40 °C M1) conditions, and the appearance, SEC, and R-CE were used as evaluation indexes to examine the stability of the formulations. The experimental results are shown in Table 12.

Experimental results show that the appearance of the 0.2-0.6 mg/mL PS80 groups is better than that of the group free of PS80 under all the examining conditions, and for the R-CE purity, the decrease in main peak of the 0.2 mg/mL PS80 group after 1 month of high temperature was lower than those of the 0.4 mg/mL and 0.6 mg/mL groups. In summary, the PS80 concentration is preferably 0.2 mg/mL.

Table 12. Experimental results of screening of surfactants for formulations

| No. | Storage conditions | Appearance | SEC% | | | R-CE% | |
|---|---|---|---|---|---|---|---|
| | | | Polymer | Monomer | △monomer | Main peak | △main peak |
| 1 | DO | Clear and transparent | 0.5 | 98.9 | | 99.3 | |
| | Shaking at 25 °C, D3 | Slightly opalescent, with a large number of particles | 0.6 | 98.9 | 0.1 | 99.2 | 0.0 |
| | 40 °CM1 | A small number of particles | 0.8 | 95.2 | 3.8 | 95.9 | 3.4 |
| 2 | DO | Clear and transparent | 0.5 | 98.9 | | 98.5 | |
| | Shaking at 25 °C, D3 | Slightly opalescent | 0.5 | 99.0 | 0.0 | 98.2 | 0.3 |
| | 40 °CM1 | Slightly opalescent | 1.0 | 94.7 | 4.2 | 95.2 | 3.3 |
| 3 | DO | Clear and transparent | 0.5 | 98.9 | | 99.2 | |
| | Shaking at 25 °C, D3 | Slightly opalescent | 0.5 | 99.0 | -0.1 | 99.0 | 0.2 |
| | 40 °CM1 | Slightly opalescent | 1.1 | 94.8 | 4.1 | 93.2 | 6.0 |
| 4 | DO | Clear and transparent | 0.5 | 98.9 | | 99.0 | |
| | Shaking at 25 °C, D3 | Slightly opalescent | 0.5 | 99.0 | -0.1 | 99.1 | -0.1 |
| | 40 °CM1 | Slightly opalescent | 1.0 | 95.3 | 3.6 | 94.0 | 5.0 |

**Example 3: Screening of Saccharides for Anti-Trop2-ADC Antibody Formulations**

Anti-Trop2-ADC antibody formulations containing 0.4 mg/mL polysorbate 80 and different types of saccharides with the protein concentration of ADC-3 being 20 mg/mL were prepared by using a 10 mM pH 6.0 His-HCl buffer system. The different saccharides are as follows, and the saccharides content is the concentration when the osmotic pressure of the solution is isotonic:

1) 80 mg/mL sucrose;
2) 88 mg/mL trehalose dihydrate;

The prepared formulations were filtered sterilely and placed in containers, which were plugged and capped. The samples were subjected to shaking (25 °C, 300 rpm, 3 days), FT5C (5 freeze-thaw cycles between -35 °C and 2-8 °C), and high temperature (40 °C M1) conditions, and the appearance, SEC, and R-CE were used as evaluation indexes to examine the stability of the formulations. The experimental results are shown in Table 13.

The experimental results show that the appearance and purity of the formulations containing sucrose and trehalose dihydrate exhibited no significant difference under different conditions.

Table 13. Experimental results of screening of saccharides

| No. | Storage conditions | Appearance | SEC% | | | R-CE% | |
|---|---|---|---|---|---|---|---|
| | | | Polymer | Monomer | △monomer | Main peak | △main peak |
| 1 | DO | Clear and transparent | 0.5 | 98.9 | | 99.2 | |
| | Shaking at 25 °C, D3 | Slightly opalescent | 0.5 | 99.0 | -0.1 | 99.0 | 0.2 |
| | FT5C | Slightly opalescent | 0.5 | 99.0 | -0.1 | 97.9 | 1.3 |
| | 40 °CM1 | Slightly opalescent | 1.1 | 94.8 | 4.1 | 93.2 | 6.0 |
| 2 | DO | Clear and transparent | 0.5 | 98.9 | | 99.1 | |
| | Shaking at 25 °C, D3 | Slightly opalescent | 0.5 | 99.0 | 0.0 | 98.3 | 0.8 |
| | FT5C | Slightly opalescent | 0.5 | 99.1 | -0.1 | 98.1 | 1.0 |
| | 40 °CM1 | Slightly opalescent | 1.1 | 95.0 | 4.0 | 93.7 | 5.4 |

**Example 4: Formula Optimization for Anti-Trop2-ADC Antibody Formulations**

Anti-Trop2-ADC antibody formulations containing 0.2 mg/mL PS80, sucrose at different concentrations, and glycine at different concentrations with the protein concentration of ADC-3 being 20 mg/mL were prepared by using a pH 6.0 His-HCl buffer system. Specific formula composition is as follows:

1) 10 mM His-HCl pH 6.0, 80 mg/mL sucrose;
2) 10 mM His-HCl pH 6.0, 40 mg/mL sucrose, 10.2 mg/mL glycine;
3) 30 mM His-HCl pH 6.0, 70 mg/mL sucrose;
4) 30 mM His-HCl pH 6.0, 40 mg/mL sucrose, 7.6 mg/mL glycine;

Two formulation modes, i.e., solution and lyophilized formulation, were used for examination.

Solutions: the prepared formulations were filtered sterilely and placed in containers, which were plugged and capped. The solutions were subjected to high temperature (40 °C D10) conditions, and the SEC and R-CE were used as evaluation indexes to examine the stability of the formulations. The experimental results are shown in Table 14. There was no significant difference in purity after storage at 40 °C D10 conditions for all groups.

Table 14. Experimental results of screening of osmotic pressure regulators for formulations

| No. | Storage conditions | Appearance | SEC% | | | R-CE% | |
|---|---|---|---|---|---|---|---|
| | | | Polymer | Monomer | △monomer | Main peak | △main peak |
| 1 | DO | Clear and transparent | 2.3 | 97.2 | N/A | 98.7 | N/A |
| | 40 °C D10 | Clear and transparent | 2.1 | 94.1 | 3.1 | 98.1 | 0.6 |
| 2 | DO | Clear and transparent | 2.2 | 97.2 | N/A | 98.7 | N/A |
| | 40 °C D10 | Clear and transparent | 2.1 | 94.2 | 3.0 | 98.0 | 0.7 |
| 3 | DO | Clear and transparent | 2.4 | 97.1 | N/A | 98.9 | N/A |
| | 40 °C D10 | Clear and transparent | 2.4 | 93.8 | 3.3 | 98.2 | 0.7 |
| 4 | DO | Clear and transparent | 2.4 | 97.0 | N/A | 98.9 | N/A |
| | 40 °C D10 | Clear and transparent | 2.5 | 93.6 | 3.5 | 98.2 | 0.7 |
| Note: in the table, "D" indicates day; for example, D10 indicates 10 days. | | | | | | | |

Lyophilized formulation: the prepared formulations were filtered sterilely, placed in containers, half-plugged, lyophilized, plugged, and capped. The appearance and the reconstitution of the lyophilized products were examined. The lyophilization procedure was as follows:

Table 15 Lyophilization procedure

| Lyophilization process parameter | Set temperature (°C) | Set time (min) | Retention time (min) | Degree of vacuum (Pa) |
|---|---|---|---|---|
| Pre-freezing | 5 | 10 | 60 | N/A |
| Pre-freezing | -45 | 50 | 120 | N/A |
| Primary drying | -20 | 120 | 3000 | 10 |
| Secondary drying | 25 | 60 | 1 | 10 |
| | 25 | 1 | 600 | 1 |

Results:

The appearance of the lyophilized powder cake showed that the bottom of the group with high sucrose concentration slightly shrank, and when the sucrose concentration was reduced to 40 mg/mL and glycine was added, the powder cake obtained under the same lyophilization conditions was uniform, full, and collapse-free, indicating that the primary drying time required when the sucrose concentration was reduced and glycine was added was shorter than that required for formulations with high sucrose concentration.

When the sucrose concentration was 40 mg/mL, samples prepared using buffers at different concentrations were uniform, full, and collapse-free in appearance after the lyophilization process.

Table 16. Results of lyophilized formulations in screening of osmotic pressure regulators

| No. | Osmotic pressure (mOsm) | Appearance after lyophilization | Reconstitution conditions |
|---|---|---|---|
| 1 | 289 | Slight edge shrinking at the bottom | Easy to reconstitute |
| 2 | 287 | White uniform cake | Easy to reconstitute |
| 3 | 279 | Slight edge shrinking at the bottom | Easy to reconstitute |
| 4 | 279 | White uniform cake | Easy to reconstitute |

**Example 5: Stability Test of Anti-Trop2-ADC Antibody Formulations**

An anti-Trop2-ADC formulation containing 40 mg/mL sucrose, 9.0 mg/mL glycine, and 0.2 mg/mL PS80 with the protein concentration of ADC-3 being 20 mg/mL were prepared by using a 30 mM pH 6.0 His-HCl buffer system.

The prepared formulation was filtered sterilely and placed in containers, which were plugged and capped. The samples were subjected to forced degradation conditions of shaking (25 °C, 300 rpm, 10 days), FT5C (5 freeze-thaw cycles between -35 °C and 2-8 °C), and high temperature (40 °C M1), acceleration at 25 °C (25 °C M3), and 2-8 °C long-term conditions (4 °C M3), and the appearance, SEC, and R-CE were used as evaluation indexes to examine the stability of the formulations. The experimental results are shown in Table 17.

The experimental results show that the appearance of the final formula formulation was clear and transparent under all the forced degradation conditions, and the final formula formulation has good stability.

Table 17. Data of stability test

| Storage conditions | Appearance | SEC% | | R-CE% | |
|---|---|---|---|---|---|
| | | Monomer | △monomer | Main peak | △main peak |
| D0 | Clear and transparent | 97.9 | N/A | 98.8 | N/A |
| Shaking for 10 days | Clear and transparent | 97.6 | 0.2 | 99.3 | -0.5 |
| FT5C | Clear and transparent | 97.6 | 0.3 | 99.3 | -0.5 |
| 40 °C D16 | Clear and transparent | 95.8 | 2.1 | 95.9 | 2.9 |
| 25 °C M3 | Clear and transparent | 94.4 | 3.5 | 96.6 | 2.3 |
| 4 °C M3 | Clear and transparent | 97.4 | 0.5 | 98.2 | 0.6 |

**Example 6**: **Lyophilization of Anti-Trop2-ADC Antibody Formulation**

An anti-Trop2-ADC antibody formulation containing 40 mg/mL sucrose, 9 mg/mL glycine, and 0.2 mg/mL PS80 with the protein concentration of ADC-3 being 20 mg/mL was prepared in the 30 mM pH 6.0 His-HCl buffer, and the formulation sample was lyophilized by using a lyophilization procedure of pre-freezing, primary drying, and secondary drying, as shown in Table 18. After the lyophilization procedure was completed, plugging was performed in vacuum. The lyophilized sample was white uniform cake in appearance, met the standards in terms of the moisture content, and was easy to reconstitute, and it met the standards for the SEC and NR-CE purity after the reconstitution. The results are shown in Table 19. The pH after reconstitution was 6.1.

Table 18. Lyophilization procedure

| Lyophilization process parameter | Set temperature (°C) | Set time (min) | Retention time (min) | Degree of vacuum (Pa) |
|---|---|---|---|---|
| Pre-freezing | 5 | 10 | 60 | N/A |
| Primary drying | -10 | 120 | 3000 | 10 |
| Secondary drying | 25 | 60 | 1 | 10 |
| | 25 | 1 | 600 | 1 |

Table 19. Data of lyophilized product

| Product appearance | Moisture content | Reconstitution time | SEC% (monomer) | NR-CE% (main peak) |
|---|---|---|---|---|
| White uniform cake | 0.8% | 1 min | 97.7% | 97.2% |

## Claims

1. A pharmaceutical composition, comprising an antibody drug conjugate and a buffer, wherein the antibody drug conjugate has a structure shown below:

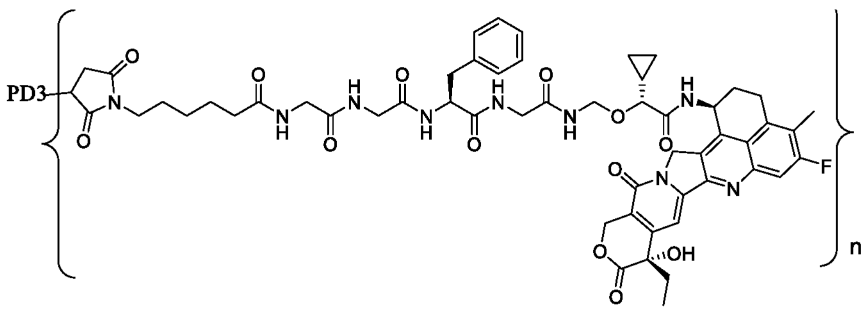

;

wherein:

PD3 is an anti-TROP2 antibody comprising a heavy chain set forth in SEQ ID NO: 1 and a light chain set forth in SEQ ID NO: 2;
n is 1 to 10, preferably 1 to 8;
the buffer of the pharmaceutical composition is a histidine-histidine hydrochloride buffer with a pH of about 5.0 to about 6.5, preferably a pH of about 5.5 to about 6.5, and
more preferably a pH of about 5.9 to about 6.2.

2. The pharmaceutical composition according to claim 1, wherein the buffer is at a concentration of about 10 mM to about 50 mM, preferably about 20 mM to about 40 mM, and more preferably about 30 mM.

3. The pharmaceutical composition according to claim 1 or 2, wherein the pharmaceutical composition further comprises a surfactant, and the surfactant is preferably a polysorbate, more preferably polysorbate 80 or polysorbate 20, and

most preferably polysorbate 80.

4. The pharmaceutical composition according to claim 3, wherein the surfactant is at a concentration of about 0.01 mg/mL to about 1.0 mg/mL, preferably about 0.1 mg/mL to about 0.3 mg/mL, and more preferably about 0.2 mg/mL.

5. The pharmaceutical composition according to any one of claims 1 to 4, wherein the pharmaceutical composition further comprises a saccharide, and the saccharide is preferably selected from the group consisting of sucrose and trehalose dihydrate and is most preferably sucrose.

6. The pharmaceutical composition according to claim 5, wherein the saccharide is at a concentration of about 25 mg/mL to about 80 mg/mL, preferably about 30 mg/mL to about 50 mg/mL, and more preferably about 40 mg/mL.

7. The pharmaceutical composition according to any one of claims 1 to 6, wherein the pharmaceutical composition further comprises an amino acid or an amino acid salt, and the amino acid or the amino acid salt is preferably selected from the group consisting of glycine and arginine hydrochloride and is more preferably glycine.

8. The pharmaceutical composition according to claim 7, wherein the glycine is at a concentration of about 6 mg/mL to about 15 mg/mL, preferably about 7 mg/mL to about 11 mg/mL, and more preferably about 9 mg/mL.

9. The pharmaceutical composition according to any one of claims 1 to 8, wherein the antibody drug conjugate is at a protein concentration of about 1 mg/mL to about 100 mg/mL, about 10 mg/mL to about 30 mg/mL, or about 18 mg/mL to about 22 mg/mL.

10. The pharmaceutical composition according to any one of claims 1 to 9, comprising the following components:

(a) the antibody drug conjugate at a protein concentration of about 10 mg/mL to about 30 mg/mL, (b) about 0.1 mg/mL to about 0.3 mg/mL polysorbate, (c) about 30 mg/mL to about 50 mg/mL sucrose, (d) about 7 mg/mL to about 11 mg/mL glycine, and (e) about 20 mM to about 40 mM histidine-histidine hydrochloride buffer, the composition having a pH of about 5.5-6.5;
wherein preferably, the pharmaceutical composition comprises the following components:
(a) the antibody drug conjugate at a protein concentration of about 18 mg/mL to about 22 mg/mL, (b) about 0.2 mg/mL polysorbate 80, (c) about 40 mg/mL sucrose, (d) about 9 mg/mL glycine, and (e) about 30 mM histidine-histidine hydrochloride buffer, the composition having a pH of about 5.9-6.2.

11. A lyophilized formulation comprising an antibody drug conjugate, wherein the lyophilized formulation can be reconstituted to form the pharmaceutical composition according to any one of claims 1 to 10.

12. A method for preparing a lyophilized formulation comprising an antibody drug conjugate, comprising a step of lyophilizing the pharmaceutical composition according to any one of claims 1 to 10.

13. A lyophilized formulation comprising an antibody drug conjugate, wherein the lyophilized formulation is obtained by lyophilizing the pharmaceutical composition according to any one of claims 1 to 10.

14. A reconstituted solution comprising an antibody drug conjugate, wherein the reconstituted solution is prepared by reconstituting the lyophilized formulation according to claim 11 or 13.

15. An article of manufacture, comprising a container, wherein the container contains the pharmaceutical composition according to any one of claims 1 to 10, the lyophilized formulation according to claim 11 or 13, or the reconstituted solution according to claim 14.

16. Use of the pharmaceutical composition according to any one of claims 1 to 10, the lyophilized formulation according to claim 11 or 13, the reconstituted solution according to claim 14, or the article of manufacture according to claim 15 in preparing a medicament for the treatment of a TROP-2-mediated disease or condition.

17. Use of the pharmaceutical composition according to any one of claims 1 to 10, the lyophilized formulation according to claim 11 or 13, the reconstituted solution according to claim 14, or the article of manufacture according to claim 15 in preparing a medicament for the treatment and/or prevention of a tumor and cancer, wherein the tumor and the cancer are preferably head and neck squamous cell carcinoma, head and neck cancer, brain cancer, neuroglioma,

glioblastoma multiforme, neuroblastoma, central nervous system carcinoma, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelium carcinoma, and Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, T-cell/histiocyte-rich large B-cell lymphoma, and lymphoplasmacytic lymphoma; the lung cancer is selected from the group consisting of non-small cell lung cancer and small cell lung cancer; the leukemia is selected from the group consisting of chronic myeloid leukemia, acute myeloid leukemia, lymphocytic leukemia, lymphoblastic leukemia, acute lymphoblastic leukemia, chronic lymphocytic leukemia, and myeloid cell leukemia.

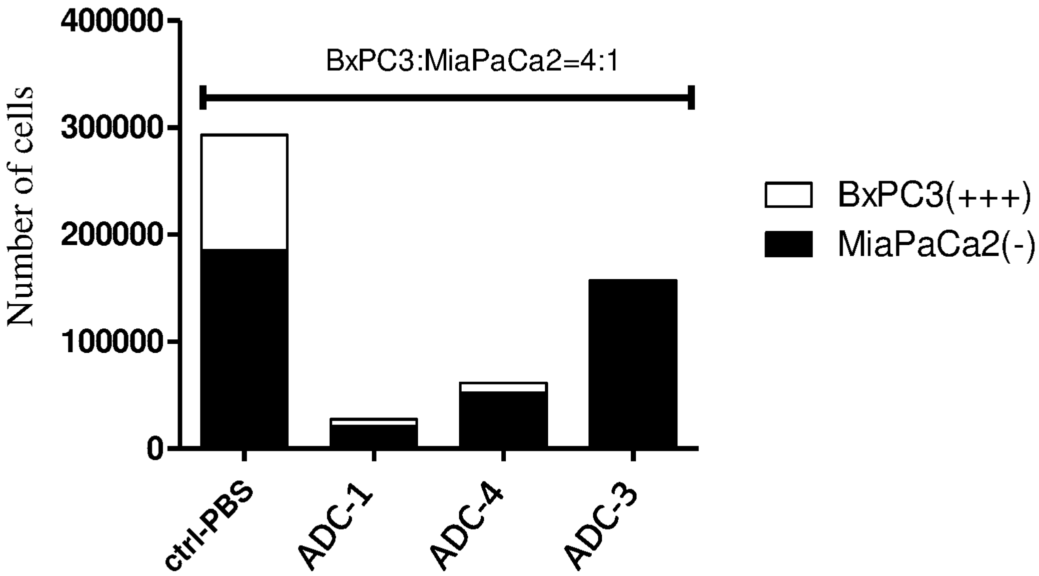
400000
300000
200000
100000
0
Number of cells
BxPC3:MiaPaCa2=4:1
BxPC3(+++)
MiaPaCa2(-)
ctrl-PBS
ADC-1
ADC-4
ADC-3

**FIG. 1**

## INTERNATIONAL SEARCH REPORT

International application No. **PCT/CN2022/107134**

**A. CLASSIFICATION OF SUBJECT MATTER**

A61K 39/395(2006.01)i; C07K 16/30(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , baidu, pudmed, STN, genbank, 中文专利序列检索系统, 江苏恒瑞医药股份有限公司, 莫希叶乐, TROP2, 偶联物, 依替喜康, exatecan, 缓冲剂, buffer, 表面活性剂, surfactant, SEQ ID NOs: 1-2, 171335-80-1/rn, 2760443-58-9/rn, 2760443-61-4/rn, 2760443-56-7/rn

**C. DOCUMENTS CONSIDERED TO BE RELEVANT**

| Category* | Citation of document, with indication, where appropriate, of the relevant passages | Relevant to claim No. |
|---|---|---|
| PY | WO 2021147993 A1 (JIANGSU HENGRUI MEDICINE CO., LTD. et al.) 29 July 2021 (2021-07-29)<br>claims 1-32 | 1-17 |
| PY | WO 2021190581 A1 (JIANGSU HENGRUI MEDICINE CO., LTD. et al.) 30 September 2021 (2021-09-30)<br>claims 1-19 | 1-17 |
| Y | CN 105849126 A (DAICHI SANKYO COMPANY, LTD. et al.) 10 August 2016 (2016-08-10)<br>description, page 65, paragraphs 942-944, page 81, paragraph 1078 | 1-17 |
| Y | WO 2020063676 A1 (JIANGSU HENGRUI MEDICINE CO., LTD. et al.) 02 April 2020 (2020-04-02)<br>description, page 98, embodiment 37, page 105, embodiment 49 | 1-17 |
| Y | WO 2021136274 A1 (BIO-THERA SOLUTIONS, LTD.) 08 July 2021 (2021-07-08)<br>claims 1-14, and description, page 16, paragraph 1 | 1-17 |
| Y | WO 2020063673 A1 (JIANGSU HENGRUI MEDICINE CO., LTD. et al.) 02 April 2020 (2020-04-02)<br>claims 1-19 | 1-17 |

☐ Further documents are listed in the continuation of Box C.   ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 September 2022** | **19 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/107134**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence listing is an XML file in Standard ST.26.

Form PCT/ISA/210 (continuation of first sheet) (January 2015)

**INTERNATIONAL SEARCH REPORT**
**Information on patent family members**

International application No.
**PCT/CN2022/107134**

| Patent document cited in search report | Publication date (day/month/year) | Patent family member(s) | Publication date (day/month/year) |
|---|---|---|---|
| WO 2021147993 A1 | 29 July 2021 | CA 3168260 A1 | 29 July 2021 |
| | | AU 2021210074 A1 | 28 July 2022 |
| | | TW 202140077 A | 01 November 2021 |
| WO 2021190581 A1 | 30 September 2021 | TW 202144013 A | 01 December 2021 |
| CN 105849126 A | 10 August 2016 | TW 202108181 A | 01 March 2021 |
| | | CN 111228510 A | 05 June 2020 |
| | | TR 201900176 T4 | 21 February 2019 |
| | | TW 201840340 A | 16 November 2018 |
| | | KR 20220009500 A | 24 January 2022 |
| | | US 2016297890 A1 | 13 October 2016 |
| | | IL 291446 A | 01 May 2022 |
| | | JP 2019069951 A | 09 May 2019 |
| | | US 2019144559 A1 | 16 May 2019 |
| | | JP 2020188801 A | 26 November 2020 |
| | | PL 3088419 T3 | 29 March 2019 |
| | | LT 3088419 T | 27 December 2018 |
| | | PH 12020551943 A1 | 07 June 2021 |
| | | SI 3088419 T1 | 31 January 2019 |
| | | KR 20160101915 A | 26 August 2016 |
| | | PH 12016501233 A1 | 15 August 2016 |
| | | KR 20200028047 A | 13 March 2020 |
| | | US 2018094073 A1 | 05 April 2018 |
| | | JP 2017197523 A | 02 November 2017 |
| | | EP 3424955 A1 | 09 January 2019 |
| | | HU E042512 T2 | 29 July 2019 |
| | | US 2021238303 A1 | 05 August 2021 |
| | | JP 2022084608 A | 07 June 2022 |
| | | KR 20210100207 A | 13 August 2021 |
| | | KR 20220068269 A | 25 May 2022 |
| | | RU 2019134399 A | 22 November 2019 |
| | | SG 11201605215 Y A | 30 August 2016 |
| | | HR P20190056 T1 | 22 February 2019 |
| | | PT 3088419 T | 11 January 2019 |
| | | CY 1121246 T1 | 29 May 2020 |
| | | JP WO2015098099 A1 | 23 March 2017 |
| | | ES 2703903 T3 | 13 March 2019 |
| | | EP 3088419 A1 | 02 November 2016 |
| | | BR 112016013704 A2 | 03 October 2017 |
| | | RS 58173 B1 | 29 March 2019 |
| | | MX 2016008192 A | 27 February 2017 |
| | | AU 2014371934 A1 | 07 July 2016 |
| | | SG 10201902571V A | 29 April 2019 |
| | | IL 267618 A | 29 August 2019 |
| | | KR 20210041105 A | 14 April 2021 |
| | | IL 246361 D0 | 31 August 2016 |
| | | CA 2933666 A1 | 02 July 2015 |
| | | WO 2015098099 A1 | 02 July 2015 |
| | | KR 20190006087 A | 16 January 2019 |
| | | DK 3088419 T3 | 28 January 2019 |
| | | AU 2020202305 A1 | 23 April 2020 |

Form PCT/ISA/210 (patent family annex) (January 2015)

**INTERNATIONAL SEARCH REPORT**
**Information on patent family members**

International application No.
**PCT/CN2022/107134**

| Patent document cited in search report | Publication date (day/month/year) | Patent family member(s) | Publication date (day/month/year) |
| --- | --- | --- | --- |
| | | TW 201609151 A | 16 March 2016 |
| | | RU 2016130095 A | 30 January 2018 |
| WO 2020063676 A1 | 02 April 2020 | CA 3114137 A1 | 02 April 2020 |
| | | JP 2022511351 A | 31 January 2022 |
| | | BR 112021004656 A2 | 01 June 2021 |
| | | EP 3858386 A1 | 04 August 2021 |
| | | KR 20210066833 A | 07 June 2021 |
| | | US 2021353764 A1 | 18 November 2021 |
| | | AU 2019349207 A1 | 08 April 2021 |
| | | TW 202027795 A | 01 August 2020 |
| | | CN 112512591 A | 16 March 2021 |
| WO 2021136274 A1 | 08 July 2021 | CN 113116812 A | 16 July 2021 |
| WO 2020063673 A1 | 02 April 2020 | JP 2022512568 A | 07 February 2022 |
| | | EP 3854816 A1 | 28 July 2021 |
| | | CN 112543771 A | 23 March 2021 |
| | | TW 202028242 A | 01 August 2020 |
| | | CA 3114474 A1 | 02 April 2020 |
| | | AU 2019351427 A1 | 15 April 2021 |
| | | KR 20210068457 A | 09 June 2021 |
| | | BR 112021004829 A2 | 08 June 2021 |
| | | US 2021347894 A1 | 11 November 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014057687 A
- CN 2021073279 W
- WO 2020063673 A
- US 20050238649 A1
- US 5208020 A
- WO 03074566 A
- WO 2015098099 A1
- WO 2020063676 A1
- WO 2015098099 A
- CN 104755494 A
- CN 105407891 A

### Non-patent literature cited in the description


- **MULLARD A.** *Nature Reviews Drug Discovery,* 2013, vol. 12, 329-332
- **DIJOSEPH JF ; ANNELLINO DC.** *Blood,* 2004, vol. 103, 1807-1814
- *Clinical Cancer Research,* 2016, vol. 22 (20), 5097-5108
- *Cancer Sci,* 2016, vol. 107, 1039-1046
- **CHARI et al.** *Cancer Research,* 1992, vol. 52, 127-131
- *J. Biol. Chem.,* 1968, vol. 243, 3558
- Antibodies: A Laboratory Manual and Molecular Cloning: A Laboratory Manual. Cold Spring Harbor Laboratory